# EUROPEAN PATENT APPLICATION

(11) **EP 4 553 154 A1**
(43) Date of publication of application: **14.05.2025**
(21) Application number: 23835616.6
(22) Date of filing: 07.07.2023
(51) Int. Cl.: C12N 5/10, C12N 15/12, C12N 15/63, C07K 14/47

(54) **MUTANT OCT3/4 PROTEIN, AND METHOD FOR PRODUCING INDUCED PLURIPOTENT STEM CELLS**

(30) Priority: 07.07.2022 JP 2022109906
(71) Applicant: RIKEN, Wako-shi, Saitama 351-0198 (JP)
(72) Inventor: HAYASHI, Yohei, Wako-shi, Saitama 351-0198 (JP); AMANO, Shinsaku, Wako-shi, Saitama 351-0198 (JP)
(74) Representative: Hoffmann Eitle
(86) International application number: PCT/JP2023/025266
(87) International publication number: WO 2024/010085

(57) **Abstract**

Provided are OCT3/4 protein variants that are capable of inducing reprogramming of somatic cells at a higher efficiency than an OCT3/4 protein consisting of a natural amino acid sequence. Also provided is a method for efficiently producing iPS cells using the OCT3/4 protein variants. The present invention provides OCT3/4 protein variants comprising an amino acid substitution, or a peptide fragment thereof comprising the amino acid substitution.

## Description

### Technical Field

The present invention relates to OCT3/4 protein variants, agents for inducing induced pluripotent stem cells, and a method for producing induced pluripotent stem cells.

### Background Art

Cell reprogramming techniques including production techniques of induced pluripotent stem cells (iPS cells) have been rapidly advanced as innovative basic techniques in bioscience, drug discovery, and regenerative medicine.

iPS cells are induced by transducing reprogramming factors such as OCT3/4, SOX2, KLF4, and C-MYC into somatic cells (Patent Literature 1 and 2; Non-Patent Literature 1 and 2). All these reprogramming factors are considered to serve as transcription factors to control the expression of a set of genes involved in self-renewal or pluripotency and thereby induce reprogramming of somatic cells.

However, the efficiency of producing iPS cells on the basis of conventional technology is extremely low. A proportion of cells where reprogramming is truly induced, among mammalian somatic cells to which reprogramming factors such as OCT3/4, SOX2, KLF4, and C-MYC are transduced, is only less than 1% (Non-Patent Literature 2).

Such a low production efficiency of iPS cells constitutes a large obstacle to clinical application. For example, when producing transplant tissues, iPS cells produced from patient's own somatic cells are preferably used from the viewpoint of immune responsiveness. However, if a production efficiency of iPS cells remains low, it will take time to prepare a sufficient number of iPS cells necessary for production of transplant tissues, and disease progression will be caused during this time. On the contrary, if the production efficiency of iPS cells can be improved, transplant tissues can be rapidly produced. Furthermore, if the number of somatic cells necessary for preparation of iPS cells is small, somatic cells collected from a patient can be decreased and a burden on the patient's body can also be reduced.

Techniques for improving safety are also explored in research for clinical applications of iPS cells. For example, a method for substituting oncogenic C-MYC with another factor is invented as a method for reducing a risk of tumorigenesis by iPS cells. However, this method extremely lowers a production efficiency of iPS cells (Non-Patent Literature 3). Improving the production efficiency of iPS cells is an important issue also in order to cover the shortcomings of such techniques for improving safety.

A common explanation for the cause of a low production efficiency of iPS cells is that the epigenetic state of somatic cells serves as a barrier that interrupts reprogramming. Thus, an approach for changing the epigenetic state of somatic cells is explored as a method for improving a production efficiency of iPS cells. A method additionally using a cytokine or a chemical substance has also been developed (Non-Patent Literature 4).

On the other hand, for a reprogramming factor itself for use for reprogramming induction, almost no attempt has been made to modify the amino acid sequence for the purpose of enhancing the function of the factor. Under the current situation, a native sequence is mainly used.

It is known that OCT3/4, which is one of the reprogramming factors, plays a leading role in reprogramming induction and pluripotency retention. A past study has reported that strict control of the activity level of OCT3/4 is important for the pluripotency retention in ES cells or the like. Non-Patent Literature 5 discloses that an increase in the expression level of OCT3/4 in ES cells induces differentiation into a primitive endoderm or a primitive mesoderm, and on the other hand, that inhibition of the activity of OCT3/4 causes the pluripotency to be lost, inducing dedifferentiation into a trophectoderm. Because of this, no attempt has hitherto been made to modify a sequence influential in the activity level of OCT3/4, or to utilize the sequence modification for reprogramming induction.

### Citation List

### Patent Literature

Patent Literature 1: WO2007/069666
Patent Literature 2: JP2008-283972A

### Non-Patent Literature

Non-Patent Literature 1: Takahashi, K. and Yamanaka, S., Cell, 2006, Aug 25; 126 (4): 663-76.
Non-Patent Literature 2: Takahashi, K. et al., Cell, 2007, Nov 30; 131 (5): 861-72.
Non-Patent Literature 3: Nakagawa, M., et al., Nat Biotechnol. 2008, Jan; 26(1): 101-6.
Non-Patent Literature 4: Takahashi, K. and Yamanaka, S., Nat Rev Mol Cell Biol. 2016, Mar; 17(3): 183-93.
Non-Patent Literature 5: Niwa, H., Miyazaki J., and Smith A.G., Nat Genet. 2000 r; 24(4): 372-6.

### Summary of Invention

### Technical Problem

An object of the present invention is to provide OCT3/4 protein variants that can induce reprogramming of somatic cells at a higher efficiency than an OCT3/4 protein consisting of a natural amino acid sequence. A method for efficiently producing iPS cells by using the OCT3/4 protein variants is also provided.

### Solution to Problem

As for the reprogramming factor OCT3/4, only a factor consisting of a natural amino acid sequence has been mainly used for reprogramming induction in the past. No attempt to modify the amino acid sequence has been made. This is because a change in the expression level of OCT3/4 causes the pluripotency of ES cells or the like to be lost (Non-Patent Literature 5), and similarly, a change in the amino acid sequence of OCT3/4 conceivably causes the reprogramming induction activity to be lost.

While such common technical knowledge is present, the present inventors have searched for OCT3/4 variants that can enhance a reprogramming efficiency by artificially modifying the amino acid sequence of the OCT3/4 protein. First, the present inventors have identified 46 amino acid residues that can directly interact with DNA in an OCT3/4 protein. Subsequently, the inventors produced a group of OCT3/4 protein variants in which one of the 46 amino acid residues is substituted with alanine, and have explored a variant having an activity of reprogramming somatic cells at a higher efficiency than a wild-type OCT3/4 protein. As a result, the inventors have found a plurality of particular substitution variants that increase the efficiency of reprogramming somatic cells, and completed the present invention. The present invention is based on the above findings, and provides the following.

(1) An OCT3/4 protein variant comprising an amino acid substitution, or a peptide fragment thereof comprising said amino acid substitution,
   wherein said amino acid substitution is a substitution of lysine at position 195 and/or glutamic acid at position 238 in the amino acid sequence of SEQ ID NO: 13.
(2) The OCT3/4 protein variant or the peptide fragment thereof of (1),
   wherein said substitution of lysine at position 195 is K195A, K195N, K195D, K195C, K195G, K195S, or K195T, and/or said substitution of glutamic acid at position 238 is E238A, E238N, E238D, E238C, E238G, or E238S.
(3) A nucleic acid encoding the OCT3/4 protein variant or the peptide fragment thereof of (1) or (2).
(4) A gene expression vector comprising the nucleic acid of (3) in a state that allows for the expression.
(5) An agent for inducing induced pluripotent stem cells (iPS cells), comprising any of the OCT3/4 protein variant or the peptide fragment thereof of (1) or (2), the nucleic acid of (3), or the gene expression vector of (4).
(6) The agent for inducing iPS cells of (5), further comprising the following (i) and/or (ii):
   (i) any of a KLF1, KLF2, KLF4, or KLF5 protein, a nucleic acid encoding any of the proteins, or a gene expression vector comprising said nucleic acid in a state that allows for the expression, or
   (ii) any of a SOX1 protein, a SOX2 protein, a SOX3 protein, a SOX15 protein or a SOX17 protein, a nucleic acid encoding any of the proteins, or a gene expression vector comprising said nucleic acid in a state that allows for the expression.
(7) The agent for inducing iPS cells of (6), further comprising the following (iii):
   (iii) any of a C-MYC protein, a T58A variant of the C-MYC protein, an N-MYC protein or an L-MYC protein, a nucleic acid encoding any of the proteins, or a gene expression vector comprising said nucleic acid in a state that allows for the expression.
(8) The agent for inducing iPS cells of (6) or (7) wherein said KLF1, KLF2, KLF4, or KLF5 protein comprises an amino acid substitution of the following (a) to (d):
   (a) serine at position 349 and/or leucine at position 356 in the amino acid sequence of SEQ ID NO: 1;
   (b) serine at position 342 and/or leucine at position 349 in the amino acid sequence of SEQ ID NO: 3;
   (c) serine at position 500 and/or leucine at position 507 in the amino acid sequence of SEQ ID NO: 5; or
   (d) serine at position 443 and/or leucine at position 450 in the amino acid sequence of SEQ ID NO: 7.
(9) The agent for inducing iPS cells of (8), wherein:
   said substitution of (a) is S349A, and/or L356A, L356N, L356D, L356C, L356E, L356G, L356K, L356M, L356S, or L356T,
   said substitution of (b) is S342A, and/or L349A, L349N, L349D, L349C, L349E, L349G, L349K, L349M, L349S, or L349T,
   said substitution of (c) is S500A, and/or L507A, L507N, L507D, L507C, L507E, L507G, L507K, L507M, L507S, or L507T, or
   said substitution of (d) is S443A, and/or L450A, L450N, L450D, L450C, L450E, L450G, L450K, L450M, L450S, or L450T.
(10) An agent for direct reprogramming, comprising any of the OCT3/4 protein variant or the peptide fragment thereof of (1) or (2), the nucleic acid of (3), or the gene expression vector of (4).
(11) Use of the agent for inducing iPS cells of any one of (5) to (9) in the production of iPS cells from somatic cells.
(12) A method for producing iPS cells, comprising
   a transduction step of transducing an agent for inducing iPS cells comprising the following (I) to (III), into somatic cells:
      (I) any of the OCT3/4 protein variant or the peptide fragment thereof of (1) or (2), the nucleic acid of (3), or the gene expression vector of (4),
      (II) any of a KLF1 protein, a KLF2 protein, a KLF4 protein, or a KLF5 protein, a nucleic acid encoding any of the proteins, or a gene expression vector comprising said nucleic acid in a state that allows for the expression, and
      (III) any of a SOX1 protein, a SOX2 protein, a SOX3 protein, a SOX15 protein or a SOX17 protein, a nucleic acid encoding any of the proteins, or a gene expression vector comprising said nucleic acid in a state that allows for the expression; and
   a culturing step of culturing the somatic cells after said transduction step in the presence of any one or more of a basic fibroblast growth factor, a TGF-β1 protein, a BMP protein, a Wnt3 protein, a GSK3β inhibitor, a Wnt inhibitor, retinoic acid, ascorbic acid, and a ROCK inhibitor.
(13) A method for producing iPS cells, comprising
   a transduction step of transducing an agent for inducing iPS cells comprising the following (I) to (IV), into somatic cells:
      (I) any of the OCT3/4 protein variant or the peptide fragment thereof of (1) or (2), the nucleic acid of (3), or the gene expression vector of (4),
      (II) any of a KLF1 protein, a KLF2 protein, a KLF4 protein, or a KLF5 protein, a nucleic acid encoding any of the proteins, or a gene expression vector comprising said nucleic acid in a state that allows for the expression,
      (III) any of a SOX1 protein, a SOX2 protein, a SOX3 protein, a SOX15 protein or a SOX17 protein, a nucleic acid encoding any of the proteins, or a gene expression vector comprising said nucleic acid in a state that allows for the expression, and
      (IV) any of a C-MYC protein, an N-MYC protein, an L-MYC protein, or a T58A variant protein of the C-MYC protein, a nucleic acid encoding any of the proteins, or a gene expression vector comprising said nucleic acid in a state that allows for the expression; and
   a culturing step of culturing the somatic cells after said transduction step.
(14) The method of (12) or (13), further comprising a selection step of selecting iPS cells transduced in said culturing step.
(15) The method of any of (12) to (14), wherein said somatic cells are derived from a human.

The present specification encompasses the disclosure of Japanese Patent Application No. 2022-109906 that serves as the basis of the priority of the present application.

### Advantageous Effects of Invention

The OCT3/4 protein variants of the present invention can induce reprogramming of somatic cells at a higher efficiency than a wild-type OCT3/4 protein consisting of a natural amino acid sequence.

### Brief Description of Drawings

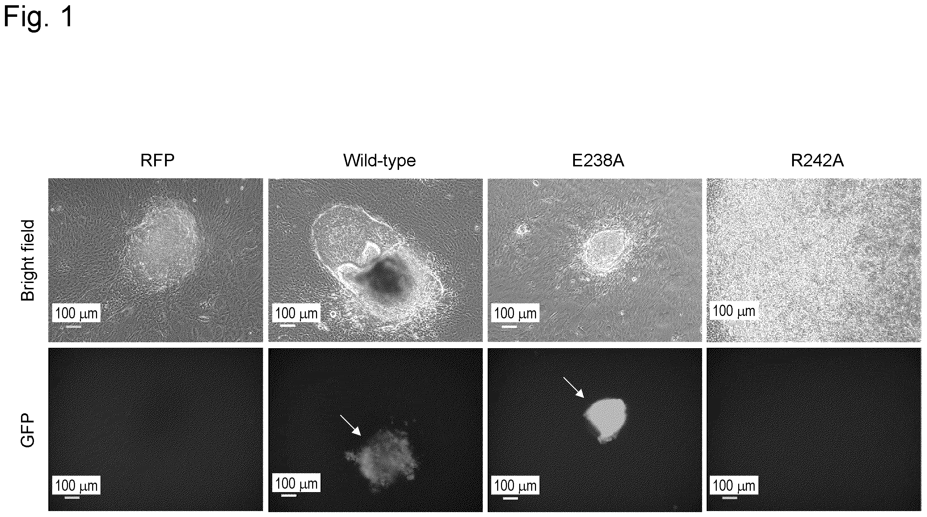

[Figure 1] Figure 1 illustrates an iPS cell colony on day 25 after the reprogramming induction of a Nanog-GFP mouse fetal fibroblast. The reprogramming induction was performed by transducing the wild-type or variant OCT3/4 gene, together with the genes encoding other reprogramming factors (KLF4, SOX2, and L-MYC), into Nanog-GFP mouse fetal fibroblasts using retroviral vectors. In the drawing, "RFP" denotes a negative control, and shows a colony of cells into which the RFP gene instead of the OCT3/4 gene was transduced together with a gene encoding another reprogramming factor. "Wild-type" shows a colony of cells into which the wild-type OCT3/4 protein was transduced. "E283A" and "R242A" show colonies of cells into which the OCT3/4 (E238A) variant and the OCT3/4 (R242A) variant were transduced respectively. In addition, "Bright field" shows a bright-field image, and "GFP" shows a fluorescence image obtained by detecting the green fluorescence of Nanog-GFP. The arrows denote the colonies in which Nanog-GFP was detected. The scale bar denotes 100 µm.

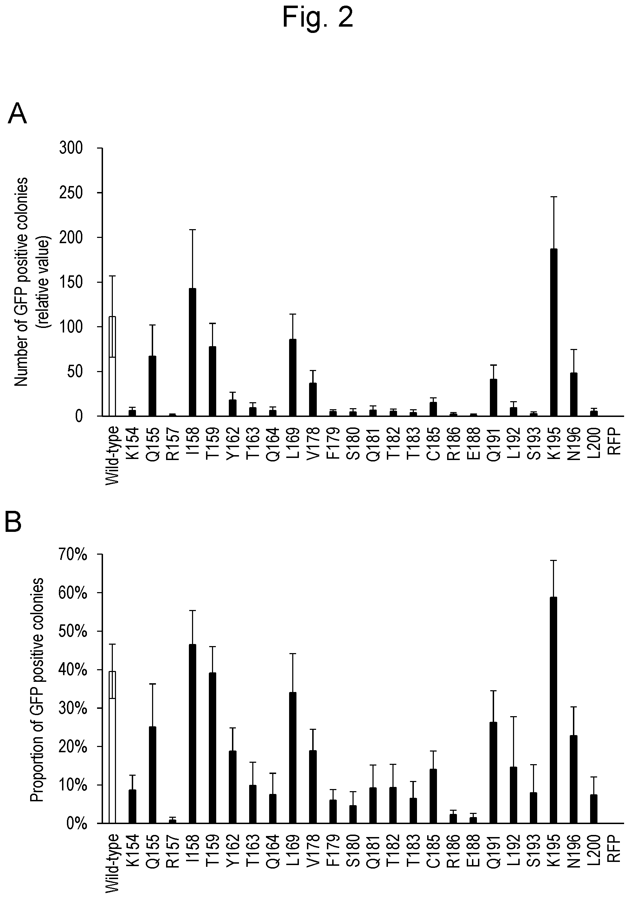

[Figure 2] Figure 2 illustrates the results of reprogramming induction using OCT3/4 protein variants in which each of the 24 amino acid residues in the POU-specific domain is substituted with alanine, in which the 24 amino acid residues are in the 46 amino acid residues capable of directly interacting with DNA. The reprogramming induction was performed by transducing the wild-type or OCT3/4 protein variant, together with other reprogramming factors (KLF4, SOX2, and L-MYC), into Nanog-GFP mouse fetal fibroblasts using retroviral vectors. Figure 2A illustrates the number of Nanog-GFP positive colonies on day 25 after viral infection, formed from 2.0 × 10⁴ Nanog-GFP mouse fetal fibroblasts into which the reprogramming factors were transduced (the number is a relative value, assuming that the number for the wild-type OCT3/4 is 100). Figure 2B illustrates the proportion of the number of Nanog-GFP positive colonies to the number of all colonies on day 25 after viral infection. The drawing shows the average of measurement data (n = 5), and each error bar represents a standard error.

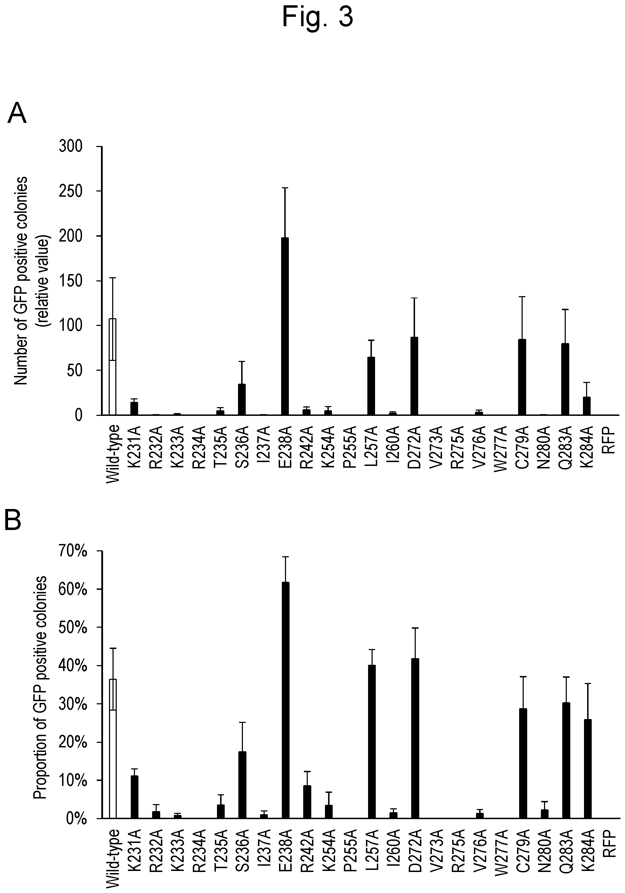

[Figure 3] Figure 3 illustrates the results of reprogramming induction using OCT3/4 protein variants in which each of the 22 amino acid residues in the POU homeodomain is substituted with alanine, in which the 22 amino acid residues are in the 46 amino acid residues capable of directly interacting with DNA. Figure 3A illustrates the number of Nanog-GFP positive colonies on day 25 after viral infection, formed from 2.0 × 10⁴ Nanog-GFP mouse fetal fibroblasts into which the reprogramming factors were transduced (the number is a relative value, assuming that the wild-type OCT3/4 is 100). Figure 3B illustrates the proportion of the number of Nanog-GFP positive colonies to the number of all colonies on day 25 after viral infection. The drawing shows the average of measurement data (n = 5), and each error bar represents a standard error.

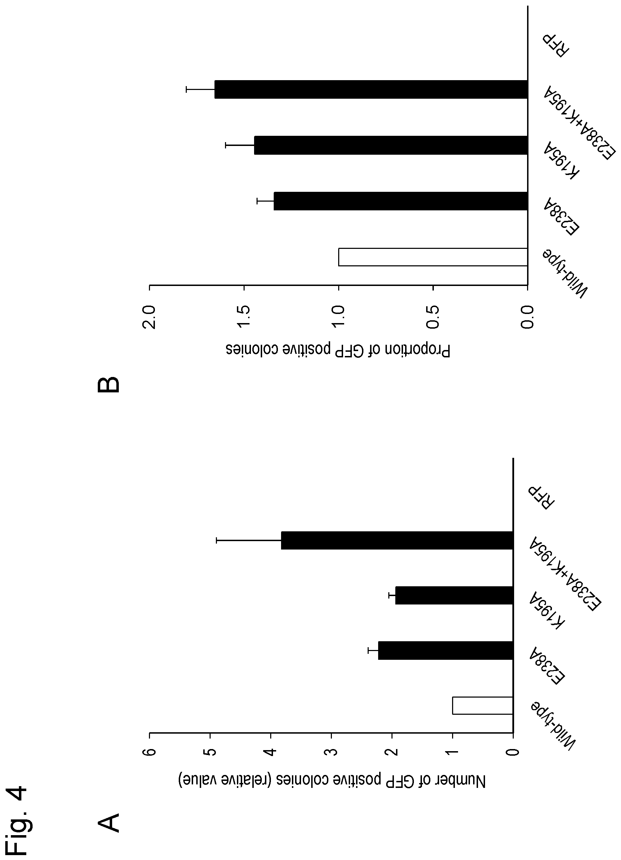

[Figure 4] Figure 4 illustrates the results of reprogramming induction, using OCT3/4 protein variants that result from undergoing introduction of any one or both of an E238A variation and a K195A variation (E238A + K196A). Figure 4A illustrates the number of Nanog-GFP positive colonies on day 25 after viral infection, formed from 2.0 × 10⁴ Nanog-GFP mouse fetal fibroblasts into which the reprogramming factors were transduced (the number is a relative value, assuming that the wild-type OCT3/4 is 1). Figure 4B illustrates the proportion of the number of Nanog-GFP positive colonies to the number of all colonies on day 25 after viral infection. The drawing shows the average of measurement data (n = 2), and each error bar represents a standard error.

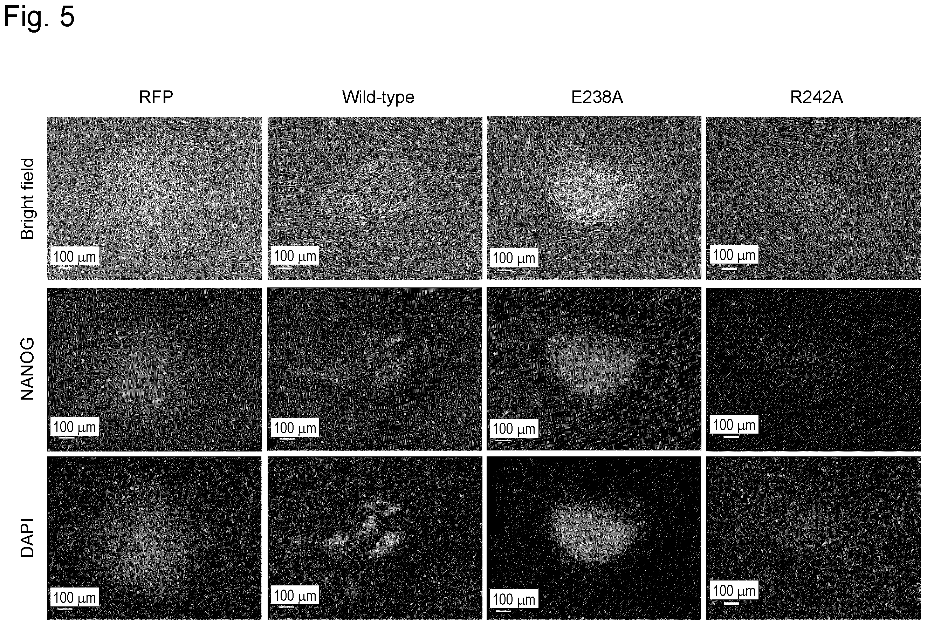

[Figure 5] Figure 5 illustrates an iPS cell colony on day 15 after the reprogramming induction of human fibroblasts. The reprogramming induction was performed by transducing the wild-type or OCT3/4 protein variant, together with other reprogramming factors (KLF4, SOX2, and L-MYC), into human fibroblasts using retroviral vectors. In the drawing, "RFP" represents a colony of cells into which no reprogramming factor was transduced, and into which only an RFP expression vector as a negative control was transduced. "Wild-type" shows cells into which the wild-type OCT3/4 protein was transduced. "E238A" and "R242A" show cells into which the OCT3/4 (E238A) variant and the OCT3/4 (R242A) variant were transduced respectively. In addition, "Bright field" shows a bright-field image. "NANOG" shows a fluorescence image obtained by detecting the Nanog protein by immunostaining. "DAPI" shows a fluorescence image obtained by detecting a nucleus by DAPI staining. The scale bar denotes 100 µm.

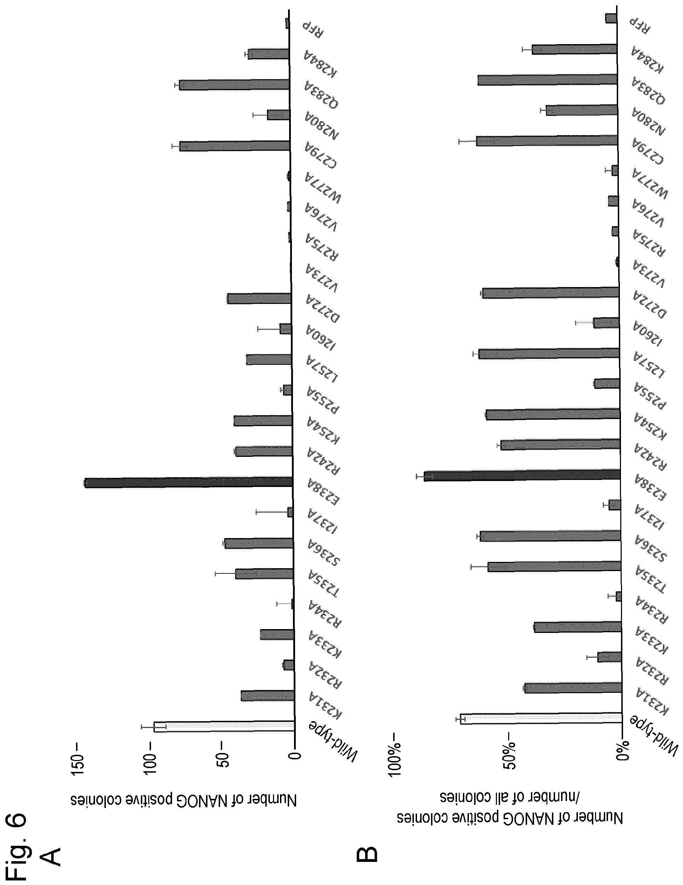

[Figure 6] Figure 6 illustrates the results of reprogramming induction using OCT3/4 protein variants in which each of the 22 amino acid residues in the POU homeodomain is substituted with alanine, in which the 22 amino acid residues are in the 46 amino acid residues capable of directly interacting with DNA. The reprogramming induction was performed by transducing the wild-type or OCT3/4 protein variant, together with other reprogramming factors (KLF4, SOX2, and L-MYC), into human fibroblasts using retroviral vectors. Figure 6A illustrates the relative value of the number of NANOG-positive colonies formed by a variant in the POU homeodomain, assuming that the number of colonies of the wild-type OCT3/4 is 100%. Figure 6B illustrates the relative value of the number of NANOG-positive colonies formed by a variant in the POU homeodomain, assuming that the number of all colonies is 100%.

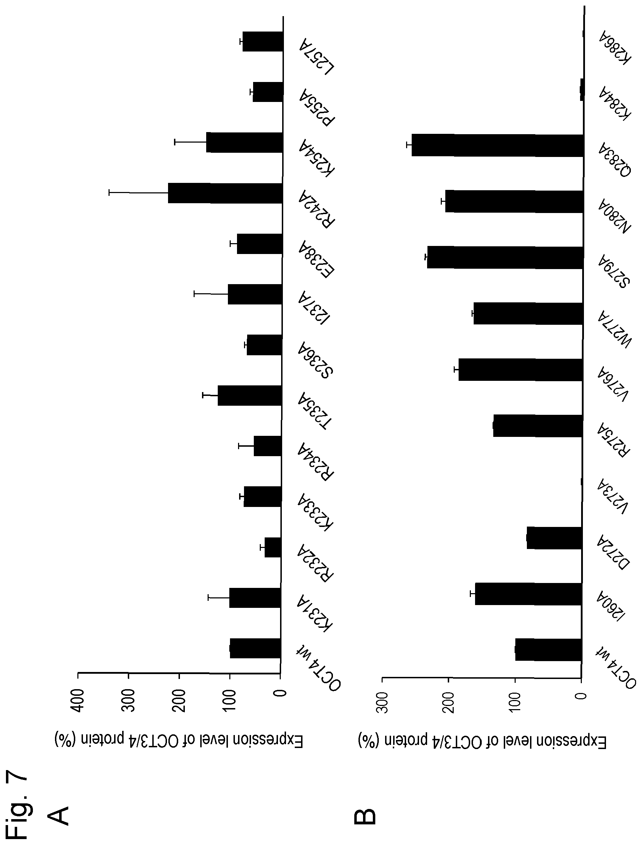

[Figure 7] Figure 7 illustrates the results of quantifying the expression level of the wild-type or OCT3/4 protein variant. Figure 7A illustrates the expression level of the wild-type OCT3/4 (OCT4 wt) protein and each OCT3/4 protein variant. Figure 7B illustrates the expression level of the wild-type OCT3/4 (OCT4 wt) protein and each OCT3/4 protein variant. The drawing shows the average of measurement data (n = 3), and each error bar represents a standard error.

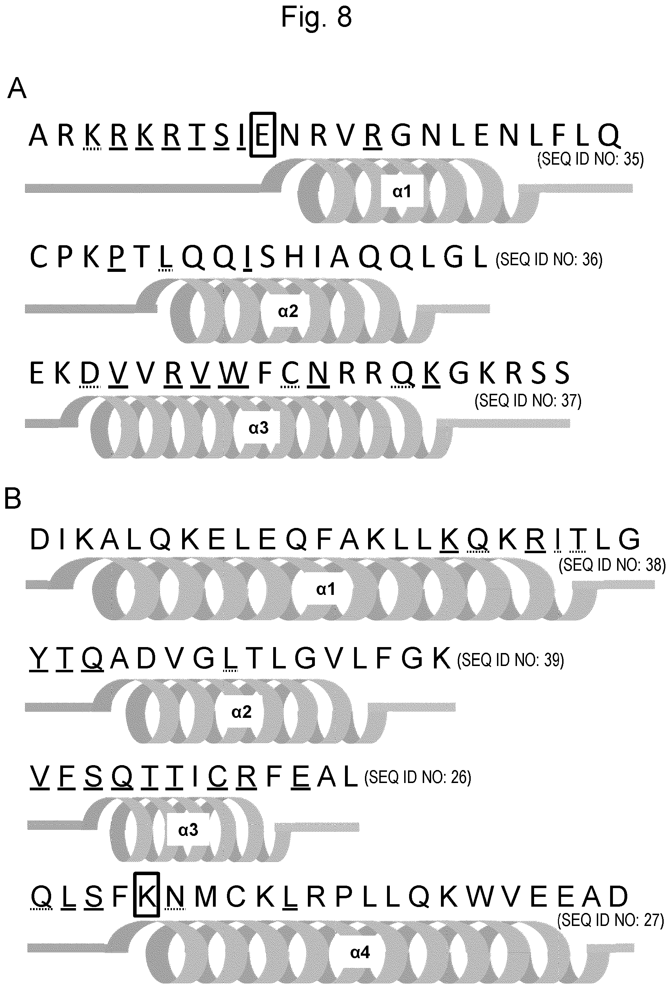

[Figure 8] Figure 8 illustrates an overview of the influence brought to the reprogramming efficiency by an amino acid substitution in the OCT3/4 protein. An amino acid residue shown in a black box represents an amino acid residue with which an alanine substitution increased the reprogramming efficiency noticeably. An amino acid residue underlined by a dotted line represents an amino acid residue with which an alanine substitution did not affect the reprogramming efficiency greatly. An amino acid residue underlined by a solid line represents an amino acid residue with which an alanine substitution decreased the reprogramming efficiency noticeably. Figure 8A illustrates the POU homeodomain (A229 to Q251 comprising α1, C252 to L269 comprising α2, and E270 to S289 comprising α3). Figure 8B illustrates the POU-specific domain (D138 to G161 comprising α1, Y162 to K177 comprising α2, V178 to L190 comprising α3, and Q191 to D212 comprising α4).

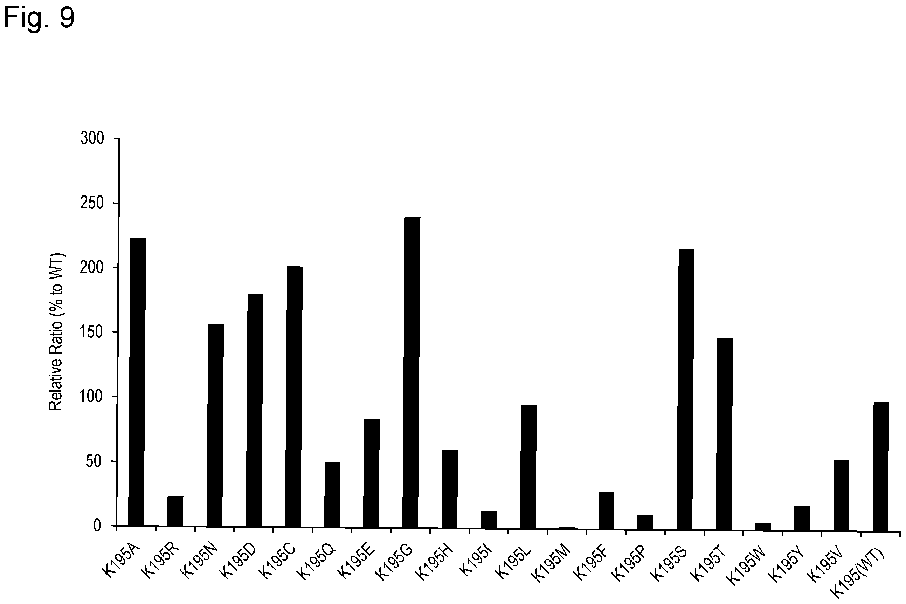

[Figure 9] Figure 9 illustrates the results of reprogramming induction using OCT3/4 protein variants and a wild-type OCT3/4 protein, each OCT3/4 protein variant resulting from an OCT3/4 protein in which the K195 site capable of increasing the efficiency of production of iPS cells were substituted with each of the 19 kinds of natural amino acid residues by a substitution variation in the DNA-binding domain. The reprogramming induction was performed by transducing the wild-type or OCT3/4 protein variant, together with other reprogramming factors (KLF4, SOX2, and L-MYC), into human fibroblasts using retroviral vectors. The number of NANOG-positive colonies formed by a K195 substitution variant is shown as a relative value, assuming that the number of colonies of the wild-type OCT3/4 is 100%.

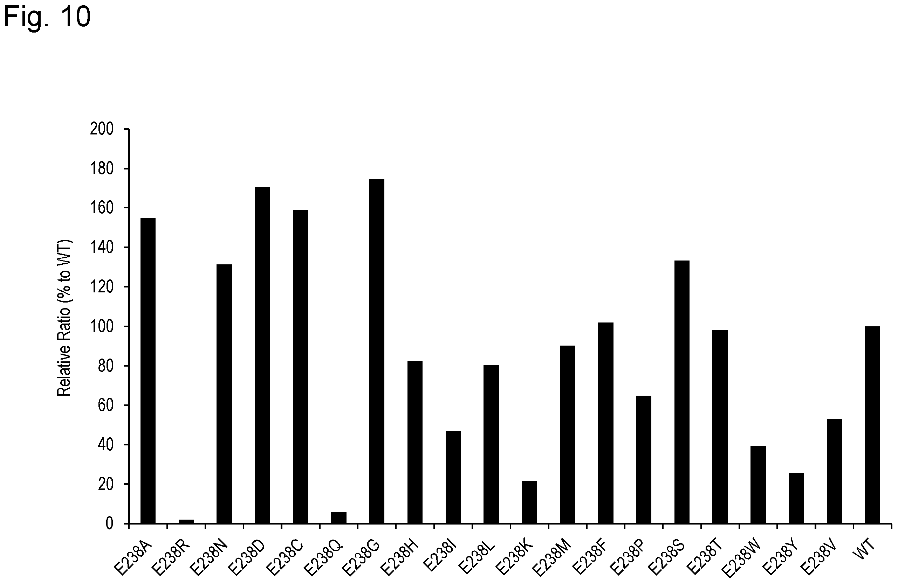

[Figure 10] Figure 10 illustrates the results of reprogramming induction using OCT3/4 protein variants and a wild-type OCT3/4 protein, each OCT3/4 protein variant resulting from an OCT3/4 protein in which the E238 site capable of increasing the efficiency of production of iPS cells were substituted with each of the 19 kinds of natural amino acid residues by a substitution variation in the DNA-binding domain. The reprogramming induction was performed by transducing the wild-type or OCT3/4 protein variant, together with other reprogramming factors (KLF4, SOX2, and L-MYC), into human fibroblasts using retroviral vectors. The number of NANOG-positive colonies formed by an E238 substitution variant is shown as a relative value, assuming that the number of colonies of the wild-type OCT3/4 is 100%.

### Description of Embodiments

### 1. OCT3/4 protein variant or peptide fragment thereof

### 1-1. Outline

A first aspect of the present invention relates to an OCT3/4 protein variant or a peptide fragment thereof. The OCT3/4 protein variant or the peptide fragment thereof of the present invention comprises a specified amino acid substitution, and transduction of it together with other reprogramming factors into somatic cells can induce reprogramming of the somatic cells at a high efficiency.

### 1-2. Definitions

The terms frequently used herein will be defined as follows.

The "OCT3/4 protein" (octamer-binding transcription factor 3/4) is a homeodomain transcription factor in the POU family, and is also referred to as POU5F1, OCT3, OCT4, or the like. The POU domain comprised in the OCT3/4 protein is a DNA-binding domain, and comprises a POU specific domain located at the N-terminal side and a POU homeodomain located at the C-terminal side. The POU specific domain and the POU homeodomain each form a helix-turn-helix structure that is one kind of DNA-binding motif. It is known that the OCT3/4 protein plays an important role in maintaining the undifferentiatedness of cells. The protein has an activity for inducing reprogramming of somatic cells when transduced into the somatic cells together with another reprogramming factor (for example, a KLF protein such as KLF4, a SOX2 protein, and a C-MYC protein). In the present invention, the OCT3/4 protein is preferably derived from a mammal. For example, the protein is derived from a mouse, rat, rabbit, bovine, cynomolgus, marmoset, or human, and is preferably derived from a human. Examples of the OCT3/4 protein include a human wild-type OCT3/4 protein consisting of the amino acid sequence of SEQ ID NO: 13 and a mouse wild-type OCT3/4 protein consisting of the amino acid sequence of SEQ ID NO: 12. In this regard, two isoforms (isoforms A and B) of the OCT3/4 protein are known. It is reported that the isoform B is an isoform not having a region consisting of 135 amino acid residues at the N-terminal side in the isoform A (the region from position 1 to position 135 in SEQ ID NO: 13), and has no reprogramming activity (Jin, W., *et al., Sci Rep.,* 2016, 6: 20818.). Because of this, the OCT3/4 protein herein means the isoform A, in principle.

The term "induced pluripotent stem cell (iPSC; iPS cell)" refers to a cell having totipotency close to that of an embryonic stem cell (ESC; ES cell) obtained from somatic cells by induction treatment. In general, iPS cells have, for example, pluripotency such that it can differentiate into any type of cells other than extraembryonic tissues in the body, and a proliferative ability such that it can proliferate almost infinitely under culturing. iPS cells can be obtained from various cells by various methods, and is usually produced by transducing four reprogramming factors of OCT3/4, SOX2, KLF4, and C-MYC proteins into somatic cells.

The term "somatic cell" herein refers to any cell other than a germ cell among cells constituting an animal individual. The somatic cell herein is not limited as long as it can achieve pluripotency by reprogramming induction. The somatic cell may be derived from any animal species. Examples of the animal species from which the somatic cell is derived include mammal species. For example, the animal species may be any mammal species such as mouse, rat, rabbit, bovine, cynomolgus, marmoset, and human, and is preferably human. A tissue or an organ from which the somatic cell is derived is not particularly limited, and is preferably a tissue or an organ that can be easily collected in which reprogramming can be efficiently induced. For example, it may be, for example, skin, an organ such as liver, blood, urine, a cancer tissue, or a pulpal cell. The somatic cells may be either differentiated cells or undifferentiated cells, or may be established cells, or primary cultured cells isolated from a tissue, and are preferably differentiated cells. Examples of the somatic cells herein include human fibroblasts, human epithelial cells, human hepatocytes, human hematocytes, mesenchymal cells, nerve cells, and muscle cells. Somatic cells for use in reprogramming induction herein are particularly referred to as "somatic cells to be reprogrammed".

The term "reprogramming" herein refers to an operation or process of changing somatic cells to another cell type. In general, the term refers to dedifferentiating a differentiated cell to change it to an undifferentiated cell. The term herein refers to an operation or process of changing somatic cells to iPS cells, unless particularly noted.

The phrase "reprogramming induction" or "induce reprogramming" herein means that reprogramming is actually achieved by applying an operation capable of triggering reprogramming, to cells. In contrast, the phrase "perform reprogramming induction" means applying an operation capable of triggering reprogramming, to cells, regardless of whether or not reprogramming is actually achieved. For example, the phrase "perform reprogramming induction" means performing an operation of transducing reprogramming factors necessary for reprogramming, into somatic cells, and culturing the somatic cell after the transduction, under a predetermined condition.

The term "pluripotency" herein has the same meaning as multipotency, and means a nature of a cell capable of differentiating into plural lineages of cells by differentiation. In particular, the term means a nature of being able to differentiate into all of endoderm, mesoderm and ectoderm, regardless of ability to differentiate into extraembryonic tissues such as placenta.

The term "reprogramming factor" herein refers to a factor that can trigger reprogramming of somatic cells by being transduced alone or together with other factor(s) into the somatic cell. When simply referring to the term "reprogramming factor" without specifying that it is, for example, a protein or a gene, it means a protein to which the reprogramming factor corresponds, a nucleic acid encoding the protein, or a gene expression vector comprising the nucleic acid. Examples of the reprogramming factor include any of four factors, OCT3/4, SOX2, KLF4, and C-MYC (herein, often referred to as "four reprogramming factors"), and an associated factor of any of said four reprogramming factors.

The term "associated factor" of any of the four reprogramming factors herein refers to a factor that can induce reprogramming of somatic cells by being transduced instead of any factor of the four reprogramming factors, into the somatic cell.

The reprogramming factor herein may be derived from any animal species. The animal species from which the reprogramming factor is derived is, but not limited to, a mammal species. For example, the animal species may be any mammal species such as mouse, rat, rabbit, bovine, cynomolgus, marmoset, or human. The animal species is preferably a human.

While the reprogramming factors and associated factors thereof will be exemplified below, the reprogramming factors and associated factors thereof herein are not limited to the following examples.

As described above, specific examples of OCT3/4 include a human OCT3/4 protein consisting of the amino acid sequence of SEQ ID NO: 13. Examples of the associated factor of OCT3/4 include NR5A2 (LRH1) and TBX3. The term "OCT3/4", as simply referred to herein, means any of an OCT3/4 protein, a gene or nucleic acid encoding the OCT3/4 protein, or a gene expression vector comprising the nucleic acid. Similarly, the term "variant OCT3/4", as simply referred to herein, means any of an OCT3/4 protein variant, a gene or nucleic acid encoding the OCT3/4 protein variant, or a gene expression vector comprising the nucleic acid.

Specific examples of KLF4 include a human KLF4 protein consisting of the amino acid sequence of SEQ ID NO: 5. Examples of the associated factor of KLF4 include KLF1, KLF2, KLF5, and the variant KLF. Examples include a human KLF1 protein consisting of the amino acid sequence of SEQ ID NO: 1, a human KLF2 protein consisting of the amino acid sequence of SEQ ID NO: 3, and a human KLF5 protein consisting of the amino acid sequence of SEQ ID NO: 7. In the case of humans, 17 kinds of KLF proteins, i.e., KLF1 to KLF17, are known. The term "KLF protein" herein means any of KLF1, KLF2, KLF4, and KLF5 proteins. It is known that KLF1, KLF2, KLF4, or KLF5 protein, when transduced together with other reprogramming factors (for example, OCT3/4, SOX2, and C-MYC proteins) into somatic cells, has an activity of inducing reprogramming of the somatic cells. In the present invention, the KLF protein is preferably derived from a mammal. For example, the protein is derived from a mouse, rat, rabbit, bovine, cynomolgus, marmoset, or human, preferably derived from a human. The term "KLF", as simply referred to herein, means any of a KLF protein, a gene or nucleic acid encoding the KLF protein, or a gene expression vector comprising the nucleic acid. Similarly, the term "variant KLF", as simply referred to herein, means any of a variant KLF protein, a gene or nucleic acid encoding the variant KLF protein, or a gene expression vector comprising the nucleic acid. The same also applies to "KLF4", "variant KLF4", and the like. In addition, the same also applies to the below-described SOX2 and C-MYC.

Specific examples of SOX2 include a human SOX2 protein consisting of the amino acid sequence of SEQ ID NO: 15. Examples of the associated factor of SOX2 include SOX1, SOX3, SOX15, SOX17, and SOX18. Examples include a human SOX1 protein consisting of the amino acid sequence of SEQ ID NO: 14, a human SOX3 protein consisting of the amino acid sequence of SEQ ID NO: 16, a human SOX15 protein consisting of the amino acid sequence of SEQ ID NO: 17, and a human SOX18 protein consisting of the amino acid sequence of SEQ ID NO: 18.

Specific examples of C-MYC include a human C-MYC protein consisting of the amino acid sequence of SEQ ID NO: 19. Examples of the associated factor of C-MYC include a T58A variant of C-MYC, N-MYC, and L-MYC. Examples include a human N-MYC protein consisting of the amino acid sequence of SEQ ID NO: 20 and a human L-MYC protein consisting of the amino acid sequence of SEQ ID NO: 21.

Other examples of the reprogramming factors and the associated factors thereof include LIN28A, LIN28B, LIN41, GLIS1, FOXH1, and HMGA2.

As a method of reprogramming somatic cells, a method of substituting some of the above reprogramming factors with a reprogramming alternative factor is also known. The term "reprogramming alternative factor" herein means a factor other than the above reprogramming factors that can trigger reprogramming when used instead of any of the above reprogramming factors. For example, a method is known in which reprogramming of somatic cells is induced using a reprogramming alternative factor instead of C-MYC among the four reprogramming factors of OCT3/4, SOX2, KLF4, and C-MYC. Specific examples of the reprogramming alternative factor that can induce reprogramming of somatic cells when used instead of C-MYC include a basic fibroblast growth factor (bFGF), a TGF-β1 protein, a BMP protein, a Wnt3 protein, a GSK3β inhibitor, a Wnt inhibitor, retinoic acid, ascorbic acid, and a ROCK inhibitor. Specific examples of the basic fibroblast growth factor (bFGF) include a human bFGF protein consisting of the amino acid sequence of SEQ ID NO: 22, specific examples of the TGF-β1 protein include a human TGF-β1 protein consisting of the amino acid sequence of SEQ ID NO: 23, specific examples of the BMP protein include a human BMP protein consisting of the amino acid sequence of SEQ ID NO: 24, specific examples of the Wnt3 protein include a human Wnt3 protein consisting of the amino acid sequence of SEQ ID NO: 25, specific examples of the GSK3β inhibitor include CHIR99021, examples of the Wnt inhibitor include IWR-1-endo, and examples of the ROCK inhibitor include Y-27632.

The term "plurality", "multiple", or "more than one" herein refers to, for example, 2 to 50, 2 to 45, 2 to 40, 2 to 35, 2 to 30, 2 to 25, 2 to 20, 2 to 15, 2 to 10, 2 to 7, 2 to 5, 2 to 4, or 2 to 3. The term "amino acid identity" refers to a proportion (%) of the number of identical amino acid residues in the total number of amino acid residues when aligning amino acid sequences of two comparative polypeptides so that the number of identical amino acid residues between them is largest with inserting a gap into one or both of the amino acid sequences as appropriate. Such alignment of the two amino acid sequences for calculation of the amino acid identity can be performed using a known program such as Blast, FASTA, or ClustalW.

The term "(amino acid) substitution" herein refers to a substitution within a group of conservative amino acids having similar properties such as charge, side chain, polarity, and aromaticity, among 20 kinds of amino acids constituting a natural protein, unless particularly noted. Examples include a substitution within a group of non-charged polar amino acids (Gly, Asn, Gln, Ser, Thr, Cys, Tyr) having a low-polar side chain, a group of branched-chain amino acids (Leu, Val, Ile), a group of neutral amino acids (Gly, Ile, Val, Leu, Ala, Met, Pro), a group of neutral amino acids (Asn, Gln, Thr, Ser, Tyr, Cys) having a hydrophilic side chain, a group of acidic amino acids (Asp, Glu), a group of basic amino acids (Arg, Lys, His), a group of aromatic amino acids (Phe, Tyr, Trp). An amino acid substitution within each of the groups is preferable because it is known that such substitution hardly changes properties of a polypeptide. However, for the OCT3/4 protein of the present invention, the amino acid substitution of lysine at position 195 or glutamic acid at position 238 in the amino acid sequence of a human wild-type OCT3/4 protein (namely, the amino acid sequence of SEQ ID NO: 13) is not limited to a substitution within a group of conservative amino acids having similar properties such as charge, side chain, polarity, and aromaticity. In addition, for the KLF protein, the amino acid substitution of serine at position 500 or leucine at position 507 in the amino acid sequence of a human wild-type KLF4 protein (namely, amino acid sequence of SEQ ID NO: 5), or the amino acid substitution at the corresponding position in another KLF protein is not limited to a substitution within a group of conservative amino acids having similar properties such as charge, side chain, polarity, and aromaticity.

### 1-3. Constitution

Constitutions of the OCT3/4 protein variant or the peptide fragment thereof, of the present invention, will be specifically described hereinafter.

The OCT3/4 protein variant of the present invention is an OCT3/4 protein variant having an activity of inducing reprogramming of somatic cells at a higher efficiency than a wild-type OCT3/4 protein.

The OCT3/4 protein variant "having an activity of inducing reprogramming of somatic cells at a higher efficiency than a wild-type OCT3/4 protein" herein means that the OCT3/4 protein variant, when transduced into somatic cells, leads to a significantly high efficiency of reprogramming induction of somatic cells as compared with a wild-type OCT3/4 protein transduced under the same conditions. For example, it means that, when transduced into somatic cells with KLF such as KLF4, SOX2, and C-MYC under the same conditions, leads to a significantly higher efficiency of reprogramming induction of somatic cells.

The term "significant" herein refers to being statistically significant. The term "statistically significant" means that there is a significant difference between measurement results of plural measuring objects in a statistical analysis of the results. In the present invention, a significant difference between measurement results from the OCT3/4 protein variant and a wild-type OCT3/4 protein in a statistical analysis of the results, corresponds to the term. Examples include a case where a risk rate (significant level) of the resulting value is low, specifically less than 5% (p < 0.05), less than 1% (p < 0.01), or less than 0.1% (p < 0.001). The "p (value)" represented here represents a probability where an assumption is accidently correct in a distribution of statistics assumed in a statistical test. Accordingly, it is meant that, as the "p'' is smaller, the assumption is more probably true. As a test method for statistical processing, a known test method capable of determining the presence or absence of significance may be appropriately used, and the test method is not particularly limited. For example, a Student t-test method or covariate variance analysis can be used.

The OCT3/4 protein variant of the present invention has an amino acid substitution at a particular position in the amino acid sequence of a wild-type OCT3/4 protein.

As a specific constitution, the OCT3/4 protein variant of the present invention comprises any amino acid substitution of lysine at position 195 and/or glutamic acid at position 238 in the amino acid sequence of the human wild-type OCT3/4 protein (namely, the amino acid sequence of SEQ ID NO: 13). More specifically, the OCT3/4 protein variant of the present invention comprises any one amino acid substitution of lysine at position 195 and glutamic acid at position 238 or both amino acid substitutions of lysine at position 195 and glutamic acid at position 238. The above OCT3/4 protein variants may be derived from a human wild-type OCT3/4 protein and comprise the above amino acid substitution in the sequence, or may be derived from a wild-type OCT3/4 protein of an animal species other than human and comprise an amino acid substitution corresponding to the above amino acid substitution, in the sequence. Examples include the mouse wild-type OCT3/4 protein that consists of the amino acid sequence of SEQ ID NO: 12, and comprises an amino acid substitution corresponding to the above-described amino acid substitution. In this regard, the substitution of lysine at position 195 and/or glutamic acid at position 238 in SEQ ID NO: 13 corresponds to the substitution of lysine at position 188 and/or glutamic acid at position 221 in SEQ ID NO: 12.

An amino acid residue after substitution in the above amino acid substitution may be any of 20 kinds of amino acid residues, namely, alanine (Ala/A) residue, cysteine (Cis/C) residue, asparagic acid (Asp/D) residue, glutamic acid (Glu/E) residue, phenylalanine (Phe/F) residue, glycine (Gly/G) residue, histidine (His/H) residue, isoleucine (Ile/I) residue, lysine (Lys/K) residue, leucine (Leu/L) residue, methionine (Met/M) residue, asparagine (Asn/N) residue, proline (Pro/P) residue, glutamine (Gln/Q) residue, arginine (Arg/R) residue, serine residue (Ser/S), threonine residue (Thr/T), valine (Val/V) residue, tryptophan (Trp/W) residue, or tyrosine (Tyr/Y) residue. The amino acid residue after substitution can be independently selected for the lysine residue at position 195 and the glutamic acid residue at position 238. For example, the glutamic acid residue at position 238 may be substituted with any of an alanine (Ala/A) residue, cysteine (Cis/C) residue, asparagic acid (Asp/D) residue, phenylalanine (Phe/F) residue, glycine (Gly/G) residue, histidine (His/H) residue, isoleucine (Ile/I) residue, lysine (Lys/K) residue, leucine (Leu/L) residue, methionine (Met/M) residue, asparagine (Asn/N) residue, proline (Pro/P) residue, glutamine (Gln/Q) residue, arginine (Arg/R) residue, serine residue (Ser/S), threonine residue (Thr/T), valine (Val/V) residue, tryptophan (Trp/W) residue, and tyrosine (Tyr/Y) residue. The lysine residue at position 195 may be substituted with any of an alanine (Ala/A) residue, cysteine (Cis/C) residue, asparagic acid (Asp/D) residue, glutamic acid (Glu/E) residue, phenylalanine (Phe/F) residue, glycine (Gly/G) residue, histidine (His/H) residue, isoleucine (Ile/I) residue, leucine (Leu/L) residue, methionine (Met/M) residue, asparagine (Asn/N) residue, proline (Pro/P) residue, glutamine (Gln/Q) residue, arginine (Arg/R) residue, serine residue (Ser/S), threonine residue (Thr/T), valine (Val/V) residue, tryptophan (Trp/W) residue, and tyrosine (Tyr/Y) residue. The amino acid residues after substitution of lysine at position 195 and glutamic acid position 238 may be the same amino acid residue, or may be amino acid residues different from each other. In a preferable embodiment, the OCT3/4 protein variant of the present invention is as follows: the amino acid substitution of lysine at position 195 in the amino acid sequence (i.e., the amino acid sequence of SEQ ID NO: 13) of the human wild-type OCT3/4 protein is K195A (substitution of a lysine residue with an alanine residue), K195N (substitution with an asparagine residue), K195D (substitution with an asparagic acid residue), K195C (substitution with a cysteine residue), K195G (substitution with a glycine residue), K195S (substitution with a serine residue), or K195T (substitution with a threonine residue); and/or the amino acid substitution of glutamic acid at position 238 is E238A (substitution of glutamic acid residue with an alanine residue), E238N (substitution with an asparagine residue), E238D (substitution with an asparagic acid residue), E238C (substitution with a cysteine residue), E238G (substitution with a glycine residue), or E238S (substitution with a serine residue). Partial examples of the OCT3/4 protein variant of the present invention include the human OCT3/4 (E238A) protein variant consisting of the amino acid sequence of SEQ ID NO: 9, the human OCT3/4 (K195A) protein variant consisting of the amino acid sequence of SEQ ID NO: 10, and the human OCT3/4 (K195A/E238A) protein variant consisting of the amino acid sequence of SEQ ID NO: 11. In this regard, "OCT3/4 (K195A/E238A)" herein means an OCT3/4 variant comprising both a K195A variation and an E238A variation.

The OCT3/4 protein variant of the present invention may comprise an addition, deletion, or substitution at a position other than the above-described positions of amino acid substitution (specifically, position 195 and position 238 in SEQ ID NO: 13). Such an OCT3/4 protein variant is preferably a polypeptide having an activity of inducing reprogramming of somatic cells at a higher efficiency than a wild-type OCT3/4 protein. For example, the OCT3/4 protein variant of the present invention is a polypeptide which consists of an amino acid sequence having an amino acid identity of 80% or more, 85% or more, 90% or more, 95% or more, 96% or more, 97% or more, 98% or more, or 99% or more to the amino acid sequence of SEQ ID NO: 13 in the entire sequence except for the above-described amino acid substitution position (specifically, position 195 and position 238 in SEQ ID NO: 13), and which has an activity of inducing reprogramming of somatic cells at a higher efficiency than a wild-type OCT3/4 protein. Alternatively, the OCT3/4 protein variant of the present invention may be a polypeptide in which one or more than one amino acids in the amino acid sequence of SEQ ID NO: 13 in the entire sequence except for the above-described amino acid substitution position (specifically, position 195 and position 238 in SEQ ID NO: 13) are deleted, substituted or added, and which has an activity of inducing reprogramming of somatic cells at a higher efficiency than a wild-type OCT3/4 protein.

The term "peptide fragment" of the OCT3/4 protein variant herein refers to a polypeptide fragment of the above-described OCT3/4 protein variant, in which the polypeptide comprises an amino acid residue substituted at any of the above-described amino acid substitution positions (specifically, position 195 and/or position 238 in SEQ ID NO: 13), and retains an activity of inducing reprogramming of somatic cells at a higher efficiency than a wild-type OCT3/4 protein (for example, the entire wild-type OCT3/4 protein). Examples include a polypeptide fragment comprising a POU domain (for example, a DNA-binding site such as a POU homeodomain or a POU-specific domain) of the OCT3/4 protein variant. The amino acid length of a polypeptide constituting the active fragment is not particularly limited. For example, the length may be a consecutive region of at least 10, 15, 20, 25, 30, 50, 100, 150, 200, 250, 300, or 350 amino acids in the OCT3/4 protein.

### 1-4. Effects

The OCT3/4 protein variant or the peptide fragment thereof of the present invention has an increased activity of the transcription factor, as compared with a wild-type OCT3/4 protein. For example, when the OCT3/4 protein variant or the peptide fragment thereof of the present invention is transduced into somatic cells, expression level of a target gene is increased, as compared with when a wild-type OCT3/4 protein is transduced, and thus a production efficiency of iPS cells is increased, as compared with a wild-type OCT3/4 protein.

The OCT3/4 protein variant or the peptide fragment thereof of the present invention can induce reprogramming of somatic cells at a higher efficiency than a wild-type OCT3/4 protein. For example, when the OCT3/4 protein variant or the peptide fragment thereof of the present invention expressed and/or purified by a known method using *Escherichia coli* or the like is directly transduced together with other reprogramming factors (for example, KLF4, SOX2, and C-MYC) into somatic cells, reprogramming of somatic cells can be induced at a higher efficiency than a case where a wild-type OCT3/4 protein is transduced together with the other reprogramming factors.

According to the OCT3/4 protein variant or the peptide fragment thereof of the present invention, iPS cells having high homogeneity can be produced. Furthermore, according to the OCT3/4 protein variant or the peptide fragment thereof of the present invention, iPS cells having low differentiation resistance (for example, iPS cells having low expression level(s) of differentiation resistance marker(s) such as HERV-H and/or lincRNA-RoR) can be produced.

### 2. Nucleic acid encoding OCT3/4 protein variant or peptide fragment thereof

### 2-1. Outline

A second aspect of the present invention relates to a nucleic acid. The nucleic acid of the present invention is a nucleic acid encoding the OCT3/4 protein variant or the peptide fragment thereof of said first aspect, for example, a DNA or an mRNA.

### 2-2. Constitution

The nucleic acid of the present invention encodes the OCT3/4 protein variant or the peptide fragment thereof of said first aspect. Examples include: a nucleic acid comprising or consisting of a nucleotide sequence identical to the nucleotide sequence of a wild-type OCT3/4 gene, except for a codon encoding the amino acid substitution of lysine at position 195 and/or glutamic acid at position 238 in the amino acid sequence of the human wild-type OCT3/4 protein; and a nucleic acid comprising or consisting of a nucleotide sequence obtained by codon optimization of the above nucleotide sequence in accordance with codon usage frequency in somatic cells into which the nucleic acid is transduced.

The nucleic acid of the present invention may be a DNA, or an RNA such as an mRNA.

Specific examples of the DNA falling into the nucleic acid of the present invention include: a DNA consisting of the nucleotide sequence of SEQ ID NO: 33, encoding an OCT3/4 protein variant where lysine at position 195 in the amino acid sequence of SEQ ID NO: 13 is substituted with alanine; a DNA consisting of the nucleotide sequence of SEQ ID NO: 32, encoding an OCT3/4 protein variant where glutamic acid at position 238 in the amino acid sequence of SEQ ID NO: 13 is substituted with alanine; and a DNA consisting of the nucleotide sequence of SEQ ID NO: 34, encoding an OCT3/4 protein variant where both lysine at position 195 and glutamic acid at position 238 in the amino acid sequence of SEQ ID NO: 13 are substituted with alanine.

The mRNA falling into the nucleic acid of the present invention is an mRNA comprising an RNA nucleotide sequence corresponding to the above DNA, as a coding region. The phrase "an RNA nucleotide sequence corresponding to the above DNA" mentioned here refers to a nucleotide sequence where thymine (T) is substituted with uracil (U) in the nucleotide sequence of the above DNA. The mRNA falling into the nucleic acid of the present invention may comprise, in addition to said coding region, a cap structure at the 5' end, a poly(A) chain at the 3' end, a 5' untranslated region (5' UTR) upstream of a start codon, and/or a 3' untranslated region (3' UTR) downstream of a stop codon. For example, 5' UTR and/or 3' UTR may comprise a sequence for regulating the amount of translation from the mRNA. For example, 3' UTR may comprise a sequence for increasing the amount of translation from an mRNA, for example, Woodchuck hepatitis virus posttranscriptional regulatory element (WPRE).

### 2-3. Effects

The DNA falling into the nucleic acid of the present invention can be used as a coding region (a protein translation region) in a gene expression vector of a third aspect.

The mRNA falling into the nucleic acid of the present invention can induce reprogramming of somatic cells at a high efficiency, for example, by directly transducing it together with mRNAs encoding other reprogramming factors, into the somatic cell. In such a case, the mRNAs are rapidly degraded after transient translation and expression of the reprogramming factors, and thus genetic factors encoding the reprogramming factors are not maintained in a cell after reprogramming. Thus, a reprogramming technique having low risk of tumorigenesis and having high safety can be provided.

### 3. Gene expression vector

### 3-1. Outline

A third aspect of the present invention relates to a gene expression vector. The gene expression vector of the present invention comprises a nucleic acid encoding the OCT3/4 protein variant or the peptide fragment thereof of the present invention, in a state that allows for the expression. According to the gene expression vector of the present invention, the OCT3/4 protein variant or the peptide fragment thereof of the present invention, can be expressed in somatic cells.

### 3-2. Constitution

### 3-2-1. Outline of constitution

The gene expression vector of the present invention comprises a promoter, and the nucleic acid described in the second aspect, as essential components.

The "gene expression vector" herein refers to a vector comprising a gene or a gene fragment in a state that allows for the expression, and comprising an expression unit capable of controlling expression of such a gene or the like. The "state that allows for the expression" herein means that a gene to be expressed is placed in a downstream region of a promoter under control of the promoter. The gene expression vector of the present invention is a vector comprising the nucleic acid of the second aspect in a state that allows for the expression, and can express the OCT3/4 protein variant or the peptide fragment thereof, in somatic cells.

Hereinafter, the vector that can be used as the gene expression vector of the present invention, and the promoter comprised in the gene expression vector of the present invention, as well as other optional components will be described.

### 3-2-2. Vector

The vector that can be used as the gene expression vector of the present invention is not particularly limited as long as it can express the OCT3/4 protein variant or the peptide fragment thereof of the present invention, in somatic cells. Examples include a viral vector, a plasmid vector, and an artificial chromosome vector.

The viral vector that can be used as the gene expression vector of the present invention is not particularly limited as long as it can infect somatic cells to be reprogrammed and can express the OCT3/4 protein variant or the peptide fragment thereof of the present invention, in the somatic cell. Examples include an adenoviral vector, an adeno-associated viral (AAV) vector, a retroviral vector, a lentiviral vector, and a Sendai virus vector. Size of a loadable DNA, types of infectable cells, cytotoxicity, the presence or absence of incorporation into host genome, an expression period, and the like are varied depending on a type of the viral vector, and can be appropriately selected depending on, for example, a type of somatic cells to be reprogrammed. For example, a replication-defective and persistent Sendai virus vector (SeVdp vector) is particularly preferable because it has a property of persistently remaining in cytoplasm without causing integration into host genome and thus has high safety (Nishimura K., et al., J Biol Chem. 2011 Feb 11; 286(6): 4760-71.; Fusaki N., et al., Proc Jpn Acad Ser B Phys Biol Sci. 2009; 85(8): 348-62.).

The plasmid vector that can be used the gene expression vector of the present invention is not particularly limited as long as it can express the OCT3/4 protein variant or the peptide fragment thereof of the present invention, in somatic cells to be reprogrammed when transduced into the somatic cell. The plasmid vector may be a shuttle vector replicable in mammal cells and bacteria such as *Escherichia coli.* A specific plasmid vector is, for example, an *Escherichia* coli-derived plasmid (pBR322, pUC18, pUC19, pUC118, pUC119, pBluescript, or the like), a streptomycete-derived plasmid (pIJ486, or the like), a Bacillus subtilis-derived plasmid (pUB110, pSH19, or the like), a yeast-derived plasmid (YEp13, YEp24, Ycp50, or the like), or a commercially available vector. Specific examples of the commercially available vector include CMV6-XL3 (OriGene Technologies), EGFP-C1, pGBT-9 (Clontech Laboratories, Inc.), pcDNA, pcDM8, and pREP4 (Thermo Fisher Scientific Inc.).

Examples of the artificial chromosome vector that can be used as the gene expression vector of the present invention include human artificial chromosome (HAC), yeast artificial chromosome (YAC), and bacterial artificial chromosome (BAC, PAC) vectors.

### 3-2-3. Promoter

The promoter comprised in the gene expression vector of the present invention is a promoter having an activity of inducing gene expression in somatic cells to be reprogrammed. Since somatic cells into which the gene expression vector of the present invention is to be transduced is, in principle, a mammal cell, in particular, a human-derived cell, the promoter may be any promoter that can express a downstream gene in such cell. Examples include a CMV promoter (CMV-IE promoter), an SV40 initial promoter, an RSV promoter, an HSV-TK promoter, an EF1α promoter, a Ub promoter, a metallothionein promoter, an SRα promoter, or a CAG promoter. Other examples also include inducible promoters such as a heatshock promoter controllable by temperature and a tetracycline-responsive promoter controllable by the presence or absence of tetracycline.

### 3-2-4. Other optional component(s)

The gene expression vector of the present invention may comprise, as optional component(s), for example, a control sequence other than the above promoter, a selection marker gene, and/or a reporter gene.

Examples of the control sequence other than the promoter, which can be encompassed in the gene expression vector of the present invention, include an expression control sequence, an intron sequence, a nuclease recognition sequence, and a replication origin sequence. Examples of the expression control sequence include expression control sequences such as an enhancer, a ribosome binding sequence, a terminator, and a poly(A) addition signal. Examples of the nuclease recognition sequence include a restriction enzyme recognition sequence, a loxP sequence recognized by a Cre recombination enzyme, a sequence targeted by artificial nuclease such as ZFN or TALEN, or a sequence targeted by a CRISPR/Cas9 system. Examples of the replication origin sequence include an SV40 replication origin sequence.

For example, the nuclease recognition sequence can be introduced upstream and downstream of the coding region of the reprogramming factor in the gene expression vector of the present invention. In this case, after completion of reprogramming of somatic cells, the nuclease can be introduced to remove the coding region of the reprogramming factor.

The selection marker gene that can be comprised in the gene expression vector of the present invention is a selection marker gene that can select somatic cells into which the gene expression vector of the present invention is transduced. Specific examples of the selection marker gene include a drug-resistant gene such as an ampicillin-resistant gene, a kanamycin-resistant gene, a tetracycline-resistant gene, a chloramphenicol-resistant gene, a neomycin-resistant gene, a puromycin-resistant gene, or a hygromycin-resistant gene.

The reporter gene that can be comprised in the gene expression vector of the present invention is a gene encoding a reporter that can distinguish somatic cells into which the gene expression vector of the present invention is transduced. Examples of the reporter gene include a gene encoding a fluorescent protein such as GFP or RFP, and a luciferase gene.

### 3-3. Effects

According to the gene expression vector of the present invention, the OCT3/4 protein variant or the peptide fragment thereof of the present invention can be expressed in somatic cells to be reprogrammed. Furthermore, reprogramming of somatic cells can be induced at a high efficiency by using the gene expression vector of the present invention in combination with other reprogramming factors (for example, KLF4, SOX2, and C-MYC).

### 4. Agent for inducing iPS cells

### 4-1. Outline

A fourth aspect of the present invention relates to an agent for inducing induced pluripotent stem cells (iPS cells). The agent for inducing iPS cells according to the present invention comprises: any of the OCT3/4 protein variant or the peptide fragment thereof, the nucleic acid encoding the OCT3/4 protein variant or the peptide fragment thereof, or the gene expression vector comprising the nucleic acid, of the present invention, as an essential component; and comprises other reprogramming factor(s), as an optional component.

### 4-2. Constitution

### 4-2-1. Outline of constitution

An agent for inducing iPS cells according to the present invention comprises, as an essential component, any of the OCT3/4 protein variant or the peptide fragment thereof of said first aspect, the nucleic acid of said second aspect, or the gene expression vector of said third aspect (hereinafter, collectively referred to as "the OCT3/4 variant of the present invention").

The agent for inducing iPS cells according to the present invention may comprise one or more other reprogramming factor(s) as optional component(s).

The essential component is according to the description of said first to third aspects, and thus only such optional component(s) will be described below.

### 4-2-2. Optional component(s)

The agent for inducing iPS cells according to the present invention may comprise (1) one or more other reprogramming factor(s), and/or (2) a reprogramming cofactor, as optional component(s). Hereinafter, (1) and (2) will be specifically described.

### (1) Other reprogramming factor(s)

The agent for inducing iPS cells according to the present invention may comprise one or more other reprogramming factor(s) as optional component(s). Reprogramming factor(s) other than the variant OCT3/4 of the present invention correspond(s) to the "other reprogramming factor(s)" here mentioned. The other reprogramming factor(s) is/are not limited as long as such factor(s) is/are reprogramming factor(s) other than the variant OCT3/4 of the present invention and can induce reprogramming of somatic cells. Examples include KLF (for example, KLF1, KLF2, KLF4, or KLF5), SOX2, C-MYC, and OCT3/4 (for example, wild-type OCT3/4) other than the variant OCT3/4 of the present invention, and an associated factor of any thereof. Examples of the associated factor include SOX1, SOX3, SOX15, SOX 17, SOX18, a T58A variant of C-MYC, N-MYC and L-MYC. These other reprogramming factors may be any of a protein corresponding to the other reprogramming factor or a peptide fragment thereof, a nucleic acid encoding said protein or the peptide fragment thereof, or a gene expression vector comprising said nucleic acid in a state that allows for the expression. Constitutions of the nucleic acid encoding the protein corresponding to the other reprogramming factor or the peptide fragment thereof, and the gene expression vector comprising said nucleic acid in a state that allows for the expression are according to the descriptions of the second aspect and third aspect described with respect to the variant OCT3/4.

The number of the other reprogramming factors comprised in the agent for inducing iPS cells according to the present invention is not limited. For example, the agent for inducing iPS cells according to the present invention may comprise, as the other reprogramming factor(s), one, two, three, four, five, six, or more reprogramming factors.

When the agent for inducing iPS cells according to the present invention comprises two or more gene expression vectors, the two or more gene expression vectors may be comprised in the same vector or may be separate vectors.

In a preferable embodiment, in addition to the essential component, the agent for inducing iPS cells according to the present invention may further comprise: any of a KLF protein (for example, a KLF 1, KLF2, KLF4, or KLF5 protein), a nucleic acid encoding the protein, or a gene expression vector comprising said nucleic acid in a state that allows for the expression; and/or any of a SOX1 protein, a SOX2 protein, a SOX3 protein, a SOX15 protein or a SOX17 protein, a nucleic acid encoding any of the proteins, or a gene expression vector comprising said nucleic acid in a state that allows for the expression. In addition, it may further comprise any of a C-MYC protein, a T58A variant of the C-MYC protein, an N-MYC protein or an L-MYC protein, a nucleic acid encoding any of the proteins, or a gene expression vector comprising said nucleic acid in a state that allows for the expression.

When the agent for inducing iPS cells according to the present invention comprises any of the following: a KLF protein (for example, a KLF 1, KLF2, KLF4, or KLF5 protein); a nucleic acid encoding the protein; and a gene expression vector comprising said nucleic acid in a state that allows for the expression, a KLF protein into which an amino acid substitution capable of increasing the reprogramming efficiency has been introduced may be used with reference to WO 2021/145128. Specifically, the KLF1, KLF2, KLF4, or KLF5 protein may comprise any amino acid substitution of the following (a) to (d):
(a) serine at position 349 and/or leucine at position 356 in the amino acid sequence of SEQ ID NO: 1;
(b) serine at position 342 and/or leucine at position 349 in the amino acid sequence of SEQ ID NO: 3;
(c) serine at position 500 and/or leucine at position 507 in the amino acid sequence of SEQ ID NO: 5; or
(d) serine at position 443 and/or leucine at position 450 in the amino acid sequence of SEQ ID NO: 7.
In another embodiment, the substitution of (a) may be S349A, and/or L356A, L356N, L356D, L356C, L356E, L356G, L356K, L356M, L356S, or L356T. In addition, the substitution of (b) may be S342A, and/or L349A, L349N, L349D, L349C, L349E, L349G, L349K, L349M, L349S, or L349T. Furthermore, the substitution of (c) may be S500A, and/or L507A, L507N, L507D, L507C, L507E, L507G, L507K, L507M, L507S, or L507T. In addition, the substitution of (d) may be S443A, and/or L450A, L450N, L450D, L450C, L450E, L450G, L450K, L450M, L450S, or L450T. WO 2021/145128 discloses that these amino acid substitutions can increase the reprogramming efficiency, compared with a wild-type KLF.

### (2) Reprogramming cofactor

The "reprogramming cofactor" that can be comprised as an optional component in the agent for inducing iPS cells according to the present invention is a factor other than those falling into the above item (1) which can increase a reprogramming induction efficiency when transduced into somatic cells but is not essential for reprogramming induction of somatic cells. Examples include NANOG, NR5A2, LIN28A, LIN28B, LIN41, GLIS1, TBX3, HMGA2, FOXH1, mir-302, mir-367, mir-106a, mir-363, an shRNA or an siRNA against TP53, dominant negative TP53, or an shRNA or an siRNA against P21.

### 4-3. Effects

The agent for inducing iPS cells according to the present invention can induce iPS cells from somatic cells.

The agent for inducing iPS cells according to the present invention can be used for producing iPS cells from somatic cells.

### 5. Agent for direct reprogramming

### 5-1. Outline

A fifth aspect of the present invention relates to an agent for direct reprogramming. The agent for direct reprogramming according to the present invention comprises, as an essential component, any of the OCT3/4 protein variant or the peptide fragment thereof, the nucleic acid encoding the OCT3/4 protein variant or the peptide fragment thereof, or the gene expression vector comprising the nucleic acid, of the present invention (hereinafter, collectively referred to as "the variant OCT3/4 of the present invention"), and comprises other direct reprogramming factor(s) as an optional component. The agent for direct reprogramming according to the present invention can directly induce other various types of cells from differentiated cells.

### 5-2. Definitions

The term "direct reprogramming (direct conversion)" herein refers to inducing, from a certain cell type, another cell type (for example, a cell type other than iPS cells) directly. More specifically, the term refers to inducing, from a certain cell type, another cell type without undergoing an iPS cell stage, for example, inducing, from differentiated cells, other various types of cells, such as nerve cells, hepatic cells, pancreatic β cells, cardiomyocytes, or endothelial cells (Ieda M., Keio JMed. 2013; 62 (3): 74-82.). The direct reprogramming herein encompasses transdifferentiation.

The term "direct reprogramming factor" herein refers to a factor that can trigger direct reprogramming by being transduced alone or together with other factor(s) into a certain cell type. The direct reprogramming factor differs depending on a type of cells subjected to direct reprogramming, and/or a type of cells induced by direct reprogramming. For example, the following is known: MyoD, which induces direct reprogramming from fibroblasts to muscle cells; a combination of Ascl1, Brn2, and Myt1l, which induces direct reprogramming from fibroblasts to nerve cells; and a combination of Gata4, Mef2c, and Tbx5, a combination of Gata4, Mef2c, Tbx5, and Hand2, a combination of Gata4, Mef2c, Tbx5, and VEGF, or a combination of Mef2c, Myocardin, and Tbx5, which induce direct reprogramming from fibroblasts to cardiomyocytes. Specific examples of direct reprogramming in which OCT3/4 is comprised as a direct reprogramming factor include; a combination of OCT3/4, SOX2, KLF4, and C-MYC, by which combination direct reprogramming is induced from fibroblasts to endothelial cells through 4-day partial reprogramming (Margariti A, et al., Proc Natl Acad Sci U S A., 2012; 109 (34): 13793-13798.); a combination of OCT3/4 and a low molecular cocktail (A83-01, thiazovivin, purmorphamine, VPA, and forskolin), by which combination direct reprogramming is induced from fibroblasts to oligodendrocyte precursor cells (Yun W., et al., NPJ Regen Med., 2022, 7 (1): 4.); a combination of OCT3/4, SOX2, KLF4, c-MYC, and SLUG, by which combination direct reprogramming is induced from urine-derived cells to nephron precursor cells (Gao, W.W., et al., Int J Mol Sci., 2021, 22 (24): 13449.); a combination of OCT3/4, SOX2, KLF4, c-MYC, and Nkx2-1, by which combination direct reprogramming is induced from fibroblasts to lung epithelial cells (Wong A.P., Sci Rep., 2019, 9 (1): 9027.); a combination of OCT3/4, SOX2, KLF4, c-MYC, and a low molecule (CHIR99021 and TTNPB), by which combination direct reprogramming is induced from fibroblasts to kidney-lineage cells (Mansoori-Moghadam, Z., et al., Exp Cell Res., 2019, 379 (2): 225-234.); a combination of OCT3/4, c-MYC, and LMP-3, by which combination direct reprogramming is induced from fibroblast to osteoblasts (Ahmed, M.F., et al., Int J Biochem Cell Biol, 2019, 106: 84-95.); and OCT3/4 by which direct reprogramming is induced from mesenchymal stromal cells to cardiomyocytes (Yannarelli G., PLoS One, 2017, 12 (12): e0189131.).

### 5-3. Constitution

The agent for direct reprogramming according to the present invention comprises, as an essential component, the variant OCT3/4 of the present invention. More specifically, it comprises, as an essential component, any of the OCT3/4 protein variant or the peptide fragment thereof of said first aspect, the nucleic acid of said second aspect, or the gene expression vector of said third aspect.

The agent for direct reprogramming according to the present invention may further comprise one or more other direct reprogramming factor(s), as optional component(s). The other direct reprogramming factor(s) comprised in the agent for direct reprogramming according to the present invention is/are varied depending on a cell type subjected to direct reprogramming and/or a cell type induced by direct reprogramming, and can be appropriately selected by those skilled in the art. Examples include, but are not limited to, KLF (for example, KLF1, KLF2, KLF4, or KLF5), SOX2, C-MYC, PAX6, VOL2, POU3F4 (BRN4), SOX9, SLUG, Nkx2-1, LMP-3, and low molecular cocktails (examples of which include A83-01, thiazovivin, purmorphamine, VPA, forskolin, CHIR99021, and/or TTNPB). The other direct reprogramming factor(s) comprised in the agent for direct reprogramming according to the present invention may be any of a protein corresponding to the other direct reprogramming factor(s), or a peptide fragment thereof, a nucleic acid encoding said protein or the peptide fragment thereof, or a gene expression vector comprising said nucleic acid in a state that allows for the expression. Constitutions of the nucleic acid encoding the protein corresponding to the other direct reprogramming factor(s) or the peptide fragment thereof, and the gene expression vector comprising said nucleic acid in a state that allows for the expression are according to the descriptions of the second aspect and third aspect described with respect to the OCT3/4 protein variant.

The number of the other direct reprogramming factor(s) comprised in the agent for direct reprogramming according to the present invention is not limited. For example, the agent for direct reprogramming according to the present invention may comprise one, two, three, four, five, six, or more other direct reprogramming factor(s), or may further comprise a low molecular cocktail comprising one or more low molecules described above.

When the agent for direct reprogramming according to the present invention comprises two or more gene expression vectors, the two or more gene expression vectors may be comprised in the same vector or may be separate vectors.

In one embodiment, the agent for direct reprogramming according to the present invention comprises KLF (for example, KLF1, KLF2, KLF4, or KLF5), SOX2, and C-MYC, in addition to the variant OCT3/4 of the present invention.

In one embodiment, the agent for direct reprogramming according to the present invention comprises a low molecular cocktail (A83-01, thiazovivin, purmorphamine, VPA, and forskolin), in addition to the variant OCT3/4 of the present invention.

In one embodiment, the agent for direct reprogramming according to the present invention comprises SOX2, KLF4, c-MYC, and SLUG, in addition to the variant OCT3/4 of the present invention.

In one embodiment, the agent for direct reprogramming according to the present invention comprises SOX2, KLF4, c-MYC, and Nkx2-1, in addition to the variant OCT3/4 of the present invention.

In another embodiment, the agent for direct reprogramming according to the present invention comprises SOX2, KLF4, c-MYC, and low molecules (CHIR99021 and TTNPB), in addition to the variant OCT3/4 of the present invention.

In one embodiment, the agent for direct reprogramming according to the present invention comprises c-MYC and LMP-3, in addition to the variant OCT3/4 of the present invention.

In one embodiment, the agent for direct reprogramming according to the present invention comprises the variant OCT3/4 of the present invention.

### 5-4. Effects

When the agent for direct reprogramming according to the present invention comprises the variant OCT3/4, KLF, SOX2, and C-MYC, for example, partial reprogramming for 4 days by the agent for direct reprogramming according to the present invention induces direct reprogramming from fibroblasts to endothelial cells at a high efficiency.

When the agent for direct reprogramming according to the present invention comprises the variant OCT3/4 and low molecular cocktails (A83-01, thiazovivin, purmorphamine, VPA, and forskolin), direct reprogramming from fibroblasts to oligodendrocyte precursor cells is induced at a high efficiency by the agent for direct reprogramming according to the present invention.

When the agent for direct reprogramming according to the present invention comprises the variant OCT3/4, SOX2, KLF4, c-MYC, and SLUG, direct reprogramming from urine-derived cells to nephron precursor cells is induced at a high efficiency by the agent for direct reprogramming according to the present invention.

When the agent for direct reprogramming according to the present invention comprises the variant OCT3/4, SOX2, KLF4, c-MYC, and Nkx2-1, direct reprogramming from fibroblasts to lung epithelial cells is induced at a high efficiency by the agent for direct reprogramming according to the present invention.

When the agent for direct reprogramming according to the present invention comprises the variant OCT3/4, SOX2, KLF4, c-MYC, and low molecules (CHIR99021 and TTNPB), direct reprogramming from fibroblasts to kidney-lineage cells is induced at a high efficiency by the agent for direct reprogramming according to the present invention.

When the agent for direct reprogramming according to the present invention comprises the variant OCT3/4, c-MYC, and LMP-3, direct reprogramming from fibroblasts to osteoblasts is induced at a high efficiency by the agent for direct reprogramming according to the present invention.

When the agent for direct reprogramming according to the present invention comprises the variant OCT3/4, direct reprogramming from mesenchymal stromal cells to cardiomyocytes is induced at a high efficiency by the agent for direct reprogramming according to the present invention.

The agent for direct reprogramming according to the present invention enables direct reprogramming from somatic cells to be performed *in vitro* or *in vivo.*

The agent for direct reprogramming according to the present invention enables another cell type to be induced without a pluripotent stem cell stage. Thus, a cell reprogramming technique having a decreased tumorigenesis risk and higher safety is provided.

### 6. iPS cell production method

### 6-1. Outline

A sixth aspect of the present invention relates to an iPS cell production method. The iPS cell production method of the present invention comprises, as essential steps, (1) an transduction step of transducing, into somatic cells, an agent for inducing iPS cells, and (2) a culturing step of culturing the somatic cell(s) after the transduction step. According to the iPS cell production method of the present invention, iPS cells can be produced at a high efficiency.

### 6-2. Method

The iPS cell production method of the present invention comprises, as essential steps, (1) an transduction step of transducing, into somatic cells, an agent for inducing iPS cells, and (2) a culturing step of culturing the somatic cell after the transduction step, and as an optional step, (3) iPS cells selection step.

Each of the transduction step and the culturing step is varied between "6-2-1. Embodiment using C-MYC or C-MYC-associated factor" and "6-2-2. Embodiment without C-MYC or C-MYC-associated factor" described below. The term "C-MYC-associated factor" herein refers to a factor which has a structure similar to C-MYC and can reprogram somatic cells by being transduced instead of C-MYC, together with other reprogramming factors, into the somatic cell (hereinafter, the same also applies to "KLF-associated factor" and "SOX2-associated factor"). Specific examples of each associated factor are as mentioned in "1-2. Definitions".

Hereinafter, each step in the above two cases will be specifically described.

### 6-2-1. Embodiment using C-MYC or C-MYC-associated factor

In the transduction step of the iPS cells production method of the present embodiment, C-MYC or a C-MYC-associated factor is transduced into somatic cells.

### (1) Transduction step

Combinations that can induce reprogramming of somatic cells, among combinations of reprogramming factors constituting the agent for inducing iPS cells in said fourth aspect, correspond to that agent for inducing iPS cells which is to be transduced into somatic cells in the transduction step in the present embodiment. That agent for inducing iPS cells which is to be transduced into somatic cells in the present embodiment may be, for example, a combination of the following factors (a) to (d): (a) any of the OCT3/4 protein variant or the peptide fragment thereof of said first aspect, the nucleic acid of said second aspect, or the gene expression vector of said third aspect (hereinafter, collectively referred to as "the variant OCT3/4 of the present invention"), (b) KLF (for example, KLF1, KLF2, KLF4, or KLF5) or a KLF-associated factor, (c) SOX2 or a SOX2-associated factor, and (d) C-MYC or a C-MYC-associated factor.

That agent for inducing iPS cells which is to be transduced into somatic cells in the present transduction step may comprise a reprogramming cofactor that can increase a production efficiency of iPS cells, in addition to the above combination of reprogramming factors. The reprogramming cofactor is according to the description of "(2) Reprogramming cofactor" in "4-2-2. Optional component(s)".

In the present transduction step, the method for transducing the agent for inducing iPS cells, into somatic cells is not limited. The transduction method may be appropriately selected depending on a type of that agent for inducing iPS cells which is to be transduced (plasmid DNA, mRNA, protein, viral vector, and the like). The agent for inducing iPS cells can be transduced into somatic cells by, for example, viral infection, a lipofection method, a liposome method, an electroporation method, a calcium phosphate method, a DEAE-Dextran method, a microinjection method, or an electroporation method. In addition, gene transduction method (transformation method) known in the art, described in Green & Sambrook, 2012, Molecular Cloning: A Laboratory Manual Fourth Ed., Cold Spring Harbor Laboratory Press, Cold Spring Harbor, New York, can be used.

### (2) Culturing step

The culturing step in the present embodiment is a step of culturing the somatic cell after said transduction step. In the present culturing step, reprogramming of somatic cells is induced by the reprogramming factors transduced in said transduction step, and iPS cells are induced. Furthermore, in the present step, whether or not iPS cells are produced can be determined by observing colony formation from the iPS cells induced.

The culturing method in the present culturing step is not limited. For example, the somatic cells after said transduction step may be cultured using feeder cells. Specific examples include: a method comprising culturing the somatic cells after said transduction step together with feeder cells in a cell culture medium; and a method comprising maintaining the somatic cells after said transduction step in a cell culture medium for 30 days to 40 days and then culturing them together with feeder cells.

The feeder cells are not limited and, for example, cells (for example, mouse fetal fibroblasts (MEFs), human fetus-derived cells, or fibroblasts), proliferation of which is stopped by radiation or antibiotic treatment, may be used.

When no feeder cell is used, a method using a culture dish covered with, for example, a basement membrane matrix, laminin, or vitronectin, or a method using a medium comprising, for example, a basement membrane matrix, laminin, or vitronectin can be used.

A known medium can be appropriately selected and then used as the cell culture medium. For example, a commercially available basal medium for mammalian cells, to which serum or a serum replacement is added, such as DMEM, may be used. For example, KnockOut (trademark) Serum Replacement: KSR (ThermoFisher, SCIENTIFIC) may be used as the serum replacement. For example, a commercially available medium for primate ES cells or primate ES/iPS cells may be used. To such a medium may be added any known additive suitable for culturing of pluripotent stem cells such as ES cells or iPS cells, for example, additive(s) such as an N2 supplement, a B27 (R) supplement, insulin, bFGF, activin A, heparin, a ROCK (Rho-associated coiled-coil forming kinase/Rho-binding kinase) inhibitor, and/or a GSK-3 inhibitor.

For the purpose of increasing a production efficiency of iPS cells, for example, TGF-β, a histone deacetylase (HDAC) inhibitor, a G9a histone methyltransferase inhibitor, and/or a p53 inhibitor may be added to the medium in the transduction step of (1) above and/or the present culturing step. As the HDAC inhibitor, for example, a low molecular inhibitor such as valproic acid (VPA), trichostatin A, sodium butyrate, MC 1293, or M344, or an siRNA against HDAC can be used, and as the G9a histone methyltransferase inhibitor, for example, a low molecular inhibitor such as BIX-01294, or an siRNA against G9a can be used, and as the p53 inhibitor, for example, a low molecular inhibitor such as Pifithrin-α, or an siRNA against p53 can be used.

Culturing conditions such as temperature, concentration of CO₂, a culturing period, and frequency of medium exchange are not limited in the present culturing step. For example, stationary culturing may be performed under 5% CO₂ at 37°C, half of the medium may be exchanged every two days, and culturing may be performed for 10 to 40 days depending on the state of colony formation.

### (3) iPS cell selection step

In the iPS cell production method of the present embodiment, the iPS cells induced after the transduction step and the culturing step may be selected.

The method for selecting the iPS cells is not limited in the present step. Examples include: a selection method using iPS cell marker gene expression as an indicator; a selection method with a selection marker gene; or a selection method with a reporter gene. The term "iPS cell marker gene" is a gene that is expressed in iPS cells but not in somatic cells to be reprogrammed. It is preferably a gene that is expressed specifically in iPS cells. For example, an Oct3/4 (for example, an endogenous Oct3/4 gene), Sox2, Nanog, ERas, Esg1, TRA1-60, or TRA-1-85 gene, and an endogenous alkaline phosphatase gene. Examples of the method for detecting expression of the iPS cell marker gene include mRNA detection methods and immunological detection methods (for example, immunostaining method, western blot method, and ELISA method). The selection marker gene and the reporter gene are according to the description of "3-2-4. Other optional component(s) in "3. Gene expression vector". In other words, it is possible to select iPS cells by selection using a drug corresponding to the selection marker gene (for example, a drug-resistant gene such as an ampicillin-resistant gene, a kanamycin-resistant gene, a tetracycline-resistant gene, a chloramphenicol-resistant gene, a neomycin-resistant gene, a puromycin-resistant gene, or a hygromycin-resistant gene) comprised in the gene expression vector transduced in said transduction step, or detection of a reporter depending on the reporter gene (for example, a gene encoding a fluorescent protein such as GFP or RFP, or a luciferase gene) comprised in the gene expression vector transduced in said transduction step.

### 6-2-2. Embodiment without C-MYC or C-MYC-associated factor

In the iPS cell production method of the present embodiment, C-MYC or C-MYC-associated factor is not transduced into somatic cells in the transduction step, and a reprogramming alternative factor is used in the culturing step. The term "reprogramming alternative factor" herein means a factor that can trigger reprogramming when used instead of the reprogramming factor as described in "1-2. Definitions". In the present embodiment, the reprogramming alternative factor is a reprogramming alternative factor such that reprogramming of somatic cells can be induced by adding the reprogramming alternative factor into a medium and performing culturing, instead of transducing C-MYC or a C-MYC-associated factor into the somatic cell. Examples include, but are not limited thereto, a basic fibroblast growth factor (bFGF), a TGF-β1 protein, a BMP protein, a Wnt3 protein, a GSK3β inhibitor, a Wnt inhibitor, retinoic acid, ascorbic acid, and a ROCK inhibitor.

### (1) Transduction step

Above combinations of reprogramming factors in "(1) Transduction step" in "6-2-1. Embodiment using C-MYC or C-MYC-associated factor" from which C-MYC or a C-MYC-associated factor is excluded, correspond to that agent for inducing iPS cells which are to be transduced into somatic cells in the transduction step in the present embodiment. Examples of that agent for inducing iPS cells which are to be transduced into somatic cells in the present embodiment include, but are not limited thereto, a combination of the following factors (a) to (c): (a) variant OCT3/4, (b) KLF (for example, KLF1, KLF2, KLF4, or KLF5) or a KLF-associated factor, and (c) SOX2 or a SOX2-associated factor.

Other conditions of the present transduction step are according to the description of "(1) Transduction step" in "6-2-1. Embodiment using C-MYC or C-MYC-associated factor" described above.

### (2) Culturing step

The culturing step in the present embodiment is a step of culturing the somatic cells after said transduction step.

In the culturing step in the present embodiment, the reprogramming alternative factor is added to a medium.

Other culturing conditions in the present culturing step are according to the description of "(2) Culturing step" in "6-2-1. Embodiment using C-MYC or C-MYC-associated factor".

### (3) iPS cell selection step

The iPS cell selection step in the present embodiment is according to the description of "(3) iPS cell selection step" in "6-2-1. Embodiment using C-MYC or C-MYC-associated factor".

### 6-3. Effects

According to the iPS cell production method of the present invention, iPS cells can be produced at a high efficiency.

According to "6-2-2. Embodiment without C-MYC or C-MYC-associated factor" in the present aspect, C-MYC or a C-MYC-associated factor being an oncogene is not transduced into somatic cells, and thus an iPS cell production method having decreased cancerization risk and higher safety is provided. It is known that a conventional method in which a wild-type OCT3/4 is used for this method is improved in safety, but on the other hand, has an extremely low production efficiency of iPS cells. However, in the present embodiment, a production efficiency of iPS cells can be improved by using the variant OCT3/4 of the present invention, and an iPS cell production method is provided in which both safety and a production efficiency are improved.

The present invention also provides a method for producing iPS cells having high homogeneity and a method for producing iPS cells having low differentiation resistance.

### Examples

### <Example 1: Search and identification of OCT3/4 variant that efficiently induces reprogramming of somatic cells at a high efficiency>

### (Purpose)

Variations are introduced into an amino acid residue capable of directly interacting with DNA to a wild-type OCT3/4 protein, and an OCT3/4 variant inducing reprogramming of somatic cells at a high efficiency is to be identified.

### (Method)

Seven complex structures of the OCT3/4 protein and DNA are registered in the Protein Data Bank (PDB), a database of steric structures of proteins (registry numbers: 7NL0, 6T93, 3L1P, 6Y0V, 6T90, 1GT0, and 6HT5). All the amino acid residues expected to directly interact with DNA in these crystal structures were identified, with the result that 22 amino acid residues in the POU homeodomain and 24 amino acid residues in the POU-specific domain were identified. These amino acid residues are illustrated as amino acid residues underlined by a dotted line, underlined by a solid line, or shown in a black box in Figure 8.

Next, 46 retroviral vectors for expressing an OCT3/4 variant and a human wild-type OCT3/4 protein were produced, wherein the OCT3/4 variant was derived from a human wild-type OCT3/4 protein consisting of the amino acid sequence of SEQ ID NO: 13 by substituting one of the 46 amino acid residues with alanine. Furthermore, retroviral vectors for expressing human wild-type KLF4 consisting of the amino acid sequence of SEQ ID NO: 5, human wild-type SOX2 consisting of the amino acid sequence of SEQ ID NO: 15, and human wild-type L-MYC consisting of the amino acid sequence of SEQ ID NO: 21, respectively, were prepared.

Specifically, a specific primer pair consisting of an approximately 25 bp nucleotide sequence for substituting a mutated site with an alanine residue was designed so that 15 bp regions comprising a mutated site were overlapped with each other. Using this primer pair, and using pMXs-hOCT3/4 (Addgene plasmid #17217) as a template, PCR was performed. In accordance with a protocol of the manufacturer, PCR was allowed in a reaction solution supplemented with 2× PCR Buffer for KOD FX Neo (TOYOBO), KOD FX Neo (TOYOBO), and 2 mM dNTPs (TOYOBO). The PCR was performed, using PCR thermal cycler (Veriti), under the following step-down cycle conditions: [94°C for 2 minutes; (98°C for 10 seconds and 74°C for 6 minutes) × 5 times; (98°C for 10 seconds and 72°C for 6 minutes) × 5 times; (98°C for 10 seconds and 70°C for 6 minutes) × 5 times; (98°C for 10 seconds and 68°C for 6 minutes) × 30 times; 68°C for 7 minutes; and then retention at 4°C].

The retroviral vectors were prepared by the following method. Plat-E cells (Cell Biolab INC, RV-101) were seeded at 3.6 × 10⁶ cells per 100 mm dish or at 5.4 × 10⁵ cells per well on a 6-well plate. On the next day, in the case of the 100 mm dish, each pMX-based retroviral vector (9 µg) for expressing human KLF4 (pMXs-hKLF4, Addgene plasmid #17219), human SOX2 (pMXs-hSOX2, Addgene plasmid #17218), human L-MYC (pMXs-Hu-L-Myc, Addgene plasmid #26022), human wild-type OCT3/4 (pMXs-hOCT3/4, Addgene plasmid #17217), or OCT3/4 variant was separately transduced into Plat-E cells using 27 µl of a FuGENE 6 transfection reagent (Promega INC, E2691). In the case of the 6-well plate, 4.5 µl of a FuGENE 6 transfection reagent and 1.5 µg of each of the retroviral vectors were used. After 24 hours, the medium was exchanged with 10 mL of DMEM comprising 10% FBS (Biosera, FB-1003/500) and P/S (Nacalai Tesque, Inc., 26253-84), and the cells were incubated for additional 24 hours. A retrovirus-comprising supernatant was collected two times after 48 hours and after 72 hours of transfection, and filtered by a cellulose acetate filter (AS ONE Corporation, 033045SO-SFCA) having a pore size of 0.45 µm. 0.5 mL each of the supernatants were mixed at equal volumes (ratio 1:1:1:1), and Polybrene (Nacalai Tesque, Inc., 12996-81) was added at a final concentration of 4 µg/mL for cell infection.

Next, mouse fetal fibroblasts were infected with the retroviral vectors, and reprogramming induction was performed. The mouse fetal fibroblasts here used were those isolated from Nanog-GFP mouse (Experimental Animal Division/RIKEN BioResource Research Center, STOCK Tg (Nanog-GFP, Puro) 1Yam, deposit No. RBRC02290) by a known method. The Nanog-GFP mouse fetal fibroblasts were infected with the retroviral vectors by subjecting 100,000 to 200,000 Nanog-GFP mouse fetal fibroblasts small in passage number to infection in a 6-well dish. The Nanog-GFP mouse fetal fibroblasts were incubated together with the retroviral vectors prepared above, for 24 hours, and then returned to a high glucose DMEM (Nacalai Tesque, Inc., 08458-16) to which a usual fibroblast culture medium (10% FBS (Biosera, FB-1003/500), P/S (Nacalai Tesque, Inc., 26253-84)) was added. The medium was exchanged at a frequency of every two days. Furthermore, 2 × 10⁴ cells were re-seeded on SL10 feeder cells (REPROCELL USA Inc) on day 6, and, on the next day, the medium was exchanged with an mES complete medium (StemSure (registered trademark) D-MEM (high glucose) (phenol red, comprising solid pyruvate) (FUJIFILM Wako Pure Chemical Corporation, 197-16275) supplemented with 15% FBS (Biosera, FB-1003/500), an MEM non-essential amino acid solution (Nacalai Tesque, Inc., 06344-56), 200 mmol/L L-Alanyl-L-glutamine Solution (100×) (Nacalai Tesque, Inc., 04260-64), StemSure (registered trademark) 50 mmol/L monothioglycerol solution (×100) (FUJIFILM Wako Pure Chemical Corporation, 195-15791), LIF (FUJIFILM Wako Pure Chemical Corporation, 129-05601), and P/S (Nacalai Tesque, Inc., 26253-84)). The medium was exchanged at a frequency of every two days and the cells were cultured for 25 days.

### (Results)

The number of colonies exhibiting green fluorescence derived from Nanog-GFP (iPS cell marker) was measured on day 25 of retroviral vector infection.

As a result, the number of Nanog-GFP positive colonies formed was reduced in most of the alanine substitution variants, as compared with wild-type OCT3/4 (Figure 2A and Figure 3A). On the contrary, the number of Nanog-GFP positive colonies formed was remarkably increased in an OCT3/4 (K195A) variant and an OCT3/4 (E238A) variant, as compared with wild-type OCT3/4 (Figure 1, Figure 2A, and Figure 3A). Furthermore, the proportion of Nanog-GFP positive colonies relative to the number of all colonies was increased in OCT3/4 (K195A) variant and the OCT3/4 (E238A) variant, as compared with wild-type OCT3/4 (Figure 2B and Figure 3B).

The increase in proportion of Nanog-GFP positive colonies indicates that proportion of fully reprogrammed iPS cells relative to partially reprogrammed iPS cells was increased. Partially reprogrammed iPS cells have low differentiation potential and pluripotency. On the contrary, fully reprogrammed iPS cells have high differentiation potential and pluripotency. Accordingly, it was demonstrated that high-quality iPS cells can be produced at a higher efficiency, and an iPS cell population having high differentiation potential and higher homogeneity can be provided by using the OCT3/4 (K195A) variant or OCT3/4 (E238A) variant in reprogramming induction.

### <Example 2: Reprogramming induction using OCT3/4 (K195A/E238A) double variant>

### (Purpose)

An OCT3/4 (K195A/E238A) double variant, which results from transduction of a K195A variation and an E238A variation both identified in Example 1, is to be produced, and a reprogramming efficiency of somatic cells is to be studied using the OCT3/4 (K195A/E238A) double variant.

### (Method and results)

A retroviral vector for expressing an OCT3/4 (K195A/E238A) double variant was produced by the following method, in which the double variant had the K195A variation and the E238A variation both found capable of increasing the reprogramming efficiency of somatic cells in Example 1, and consisted of the amino acid sequence of SEQ ID NO: 11. Specifically, a specific primer pair consisting of an approximately 25 bp nucleotide sequence for substituting a lysine (K) residue at position 195 with an alanine residue was designed so that 15 bp regions comprising a mutated site were overlapped with each other. PCR was performed, using this primer pair, and using, as a template, a pMXs-hOCT3/4 having the E238A variation introduced therein, in which the variation was produced in Example 1. The PCR was performed by the same method as in Example 1.

Together with the retroviral vector for expressing the OCT3/4 (K195A/E238A) double variant, retroviral vectors for expressing KLF4, SOX2, and L-MYC were allowed to infect a mouse fetal fibroblast to perform reprogramming induction. The specific conditions for reprogramming induction were in accordance with the method described in Example 1. In this Example, a wild-type OCT3/4, an OCT3/4 (K195A) single variant, and an OCT3/4 (E238A) single variant were used as comparative controls.

The number of colonies exhibiting green fluorescence derived from Nanog-GFP (iPS cell marker) was measured on day 25 of retroviral vector infection. The results are shown in Figure 4. The number of Nanog-GFP positive colonies formed was remarkably increased in the OCT3/4 (K195A/E238A) double variant, as compared with the OCT3/4 (K195A) single variant and OCT3/4 (E238A) single variant (Figure 4A). Furthermore, the proportion of Nanog-GFP positive colonies relative to the number of all colonies was also remarkably increased in the OCT3/4 (K195A/E238A) double variant, as compared with the OCT3/4 (K195A) single variant and OCT3/4 (E238A) single variant (Figure 4B).

From the result, it was demonstrated that it is possible to produce a higher-quality iPS cell at a higher efficiency with the OCT3/4 (K195A/E238A) double variant, as compared with the OCT3/4 (K195A) single variant and the OCT3/4 (E238A) single variant.

### <Example 3: iPS cell production from normal human fibroblast using OCT3/4 variant>

### (Purpose)

Reprogramming induction was performed on a normal human fibroblast using an OCT3/4 variant produced by substituting, with alanine, one of the 22 amino acid residues capable of directly interacting with DNA in the POU homeodomain. This allows identifying an OCT3/4 variant capable of inducing reprogramming of somatic cells in a normal human fibroblast at a high efficiency.

### (Method)

In this Example, the 22 amino acid residues substituted in the POU homeodomain in Example 1 (the amino acid residues underlined by a dotted line, underlined by a solid line, or shown in a black box in Figure 8A) were substituted with alanine.

Normal human fibroblasts (NB1RGB cells) were cultured in a Dulbecco's modified eagle medium (4.5 g/L glucose, Nacalai Tesque, Inc., 08458-16) comprising 10% FBS (Biosera, FB-1003/500), P/S, penicillin (100 units/mL) and streptomycin (100 µg/mL) (Nacalai Tesque, Inc., 26253-84).

Reprogramming induction of normal human fibroblasts was performed by the following method. A total of five types of vectors were transfected into Plat-GP (Cell Biolab INC, RV-103) cells using a FuGENE 6 transfection reagent in such a manner that the ratio of each of the pMX vectors comprising human KLF4 (Addgene plasmid #17219), human SOX2 (Addgene plasmid #17218), human L-MYC (Addgene plasmid #26022), and human wild-type OCT3/4 (pMXs-OCT3/4, Addgene plasmid #17217) or OCT3/4 variant to a pCMV-VSV-G virus envelope vector (RDB04392, RIKEN BRC lentiviral vector plasmid) was 3:1. The resultant virus supernatant was used for viral infection of 2 × 10⁴ NB1RGB cells small in passage number, in a 6-well dish overnight.

On day 20 after viral infection, a NANOG protein was fluorescently-labeled using an immunofluorescent staining method. Colonies were observed under a fluorescence microscope, and the number of NANOG positive colonies was measured.

### (Results)

The results of measuring the number of NANOG positive colonies, and calculating a relative value, assuming that the number of all colonies is 100%, are shown in Figure 6. The number of NANOG positive colonies formed was reduced in most of the alanine substitution variants, as compared with wild-type OCT3/4 (Figure 6). On the contrary, the number of NANOG positive colonies was remarkably increased in the OCT3/4 (E238A) variant, as compared with the wild-type OCT3/4 (Figure 5 and Figure 6).

The above results agreed with the results in Examples 1 and 2 in which a mouse fetal fibroblast was used.

The position of the substitution variation and the effect thereof in the OCT3/4 protein, which were studied in Example 1 and this Example, are shown in Figure 8A (the POU homeodomain) and Figure 8B (the POU specific domain). In Figure 8, the amino acid residues shown in a black box show the amino acid residues remarkably increased in the reprogramming efficiency by alanine substitution; the amino acid residues underlined by a dotted line show the amino acid residues not changed significantly in the reprogramming efficiency by alanine substitution; and the amino acid residues underlined by a solid line show the amino acid residues remarkably decreased in the reprogramming efficiency by alanine substitution.

### <Example 4: Influence on expression level of OCT3/4 protein>

### (Purpose)

Influence of alanine substitution introduction on the expression level of an OCT3/4 protein is studied.

### (Method)

Using the same method as in Example 1, a retroviral vector for transducing each kind of variant OCT3/4 was allowed to infect a Nanog-GFP mouse fetal fibroblast. Specifically, with respect to 100,000 Nanog-GFP mouse fetal fibroblasts having a small passage number, the Nanog-GFP mouse fetal fibroblasts together with retroviral vectors prepared by the same method as in Example 1 were incubated in a 6-well dish for 24 hours. Then, the medium was exchanged with a high glucose DMEM (Nacalai Tesque, Inc., 08458-16) supplemented with a usual fibroblast culture medium (10% FBS (Biosera, FB-1365/500), P/S (Nacalai Tesque, Inc., 26253-84)). On day 3 after infection, cells were detached, using 200 µL of liquid obtained by mixing Sample Buffer Solution without 2-ME (2X) for SDS-PAGE (Nacalai tesque, 30567-12) and 1 mol/L Dithiothreitol Solution (Nacalai tesque, 14130-41) at a ratio of 9:1, and using a cell scraper (Cell Scraper for MICROPLATE, Dish Sterile 100 pcs (IWAKI, 9000-220)). The cells were heated at 95°C for 5 minutes to prepare a sample.

The amount of the OCT3/4 protein comprised in the resulting sample was analyzed in accordance with a protocol of the manufacturer, using a SimpleWestem system (simple wes, Protein Simple Inc.). Specifically, 40 µL of water was added to a DTT tube. To a 5× Fluorscent Master Mix tube, 20 µL of a 10× Sample Buffer2 & DTT solution was added. To a Biotin Ladder tube, 20 µL of water was added. The resulting solution was pipetted and left to stand on ice. To a 1.5 mL tube, 1.2 µL of a 10× Sample Buffer2 and 118.8 µL of water were added. To each 0.2 mL tube, 9 µL of the diluted Sample buffer2 solution and 1 µL of each protein sample were added. To each 0.2 mL tube, 2.5 µL of the diluted DTT solution was added. Antibody Diluent II in an amount of 150 µL and Anti-h/m/rGAPDH/G3PDH [R&D]H in an amount of 0.15 µL were mixed. Milk-Free Antibody Diluent in an amount of 142.5 µL and Anti-h/mOCT3/4 [R&D systems; AF1759] in an amount of 7.5 µL were mixed. The reagent, sample, and antibody prepared as above, and water were introduced into a microplate and then analyzed.

### (Results)

The results of quantifying the protein expression level of each alanine substitution variant are shown in Figure 7. With the OCT3/4 (E238A) protein found capable of increasing the reprogramming efficiency of somatic cells in the above Example, no significant change in the expression level was detected, as compared with the wild-type OCT3/4 protein (Figure 7A). Accordingly, it was suggested that the increase caused in the reprogramming efficiency of somatic cells by OCT3/4 (E238A) is not due to the increase in the protein expression level, but due to the augmentation of the activity.

### <Example 5: iPS cell production from normal human fibroblast using OCT3/4 variant>

### (Purpose)

In the DNA-binding domain of an OCT3/4 protein, the K195 site and the E238 site capable of increasing the efficiency of production of iPS cells by a substitution variation is to bel each substituted with 19 kinds of natural amino acid residues. Using a total of 38 OCT3/4 variants and wild-type OCT3/4, reprogramming induction was performed on a normal human fibroblast. This allows for identifying an OCT3/4 variant capable of inducing reprogramming of somatic cells in a normal human fibroblast at a high efficiency.

### (Method)

The subjects of this Example were a total of 38 kinds of OCT3/4 variants and a wild-type OCT3/4, in each of which variants the K195 site or the E238 site found in Examples 1 and 3, and capable of increasing the efficiency of production of iPS cells was substituted with each of 19 kinds of natural amino acid residues.

Normal human fibroblasts (NB1RGB cells) were cultured in a Dulbecco's modified eagle medium (4.5 g/L glucose, Nacalai Tesque, Inc., 08458-16) comprising 10% FBS (Biosera, FB-1003/500), P/S, penicillin (100 units/mL) and streptomycin (100 µg/mL) (Nacalai Tesque, Inc., 26253-84).

Reprogramming induction of normal human fibroblasts was performed by the following method. A total of five types of vectors were transfected into Plat-GP (Cell Biolab INC, RV-103) cells using a FuGENE 6 transfection reagent in such a manner that the ratio of each of the pMX vectors encoding human KLF4 (Addgene plasmid #17219), human SOX2 (Addgene plasmid #17218), human L-MYC (Addgene plasmid #26022), and human wild-type OCT3/4 (pMXs-OCT3/4, Addgene plasmid #17217) or OCT3/4 variant to a pCMV-VSV-G virus envelope vector (RDB04392, RIKEN BRC lentiviral vector plasmid) was 3:1. The resultant virus supernatant was used for viral infection of 2 × 10⁴ NB1RGB cells small in passage number, in a 12-well dish overnight.

On day 20 after viral infection, a NANOG protein was fluorescently-labeled using an immunofluorescent staining method. Colonies were observed under a fluorescence microscope, and the number of NANOG positive colonies was measured.

### (Results)

The results of counting the number of NANOG positive colonies, and calculating a relative value, assuming that the number of wild-type OCT3/4 colonies is 100%, are shown in Figure 9 and Figure 10. Among the K195 substitution variants, each variant of K195A, K195N, K195D, K195C, K195G, K195S, and K195T demonstrated that the number of NANOG positive colonies formed was increased, as compared with the wild-type OCT3/4 (Figure 9). Among the E238 substitution variants, K238A, E238N, E238D, E238C, E238G, and E238S demonstrated that the number of NANOG positive colonies formed was increased, as compared with the wild-type OCT3/4 (Figure 10). From this result, it was demonstrated that, with the OCT3/4 variant, iPS cells are produced at an extremely high efficiency by suitably substituting any one of the K195 site and the E238 site.

All publications, patents, and patent applications cited herein are incorporated herein by reference in their entirety.

## Claims

1. An OCT3/4 protein variant comprising an amino acid substitution, or a peptide fragment thereof comprising said amino acid substitution,
wherein said amino acid substitution is a substitution of lysine at position 195 and/or glutamic acid at position 238 in the amino acid sequence of SEQ ID NO: 13.

2. The OCT3/4 protein variants or the peptide fragment thereof of claim 1,
wherein said substitution of lysine at position 195 is K195A, K195N, K195D, K195C, K195G, K195S, or K195T, and/or said substitution of glutamic acid at position 238 is E238A, E238N, E238D, E238C, E238G, or E238S.

3. A nucleic acid encoding the OCT3/4 protein variant or the peptide fragment thereof of claim 1.

4. A gene expression vector comprising the nucleic acid of claim 3 in a state that allows for the expression.

5. An agent for inducing induced pluripotent stem cells (iPS cells), comprising any of the OCT3/4 protein variant or the peptide fragment thereof of claim 1, the nucleic acid of claim 3, or the gene expression vector of claim 4.

6. The agent for inducing iPS cells of claim 5, further comprising the following (i) and/or (ii):
(i) any of a KLF1 protein, a KLF2 protein, a KLF4 protein, or a KLF5 protein, a nucleic acid encoding any of the proteins, or a gene expression vector comprising said nucleic acid in a state that allows for the expression, or
(ii) any of a SOX1 protein, a SOX2 protein, a SOX3 protein, a SOX15 protein or a SOX17 protein, a nucleic acid encoding any of the proteins, or a gene expression vector comprising said nucleic acid in a state that allows for the expression.

7. The agent for inducing iPS cells of claim 6, further comprising the following (iii):
(iii) any of a C-MYC protein, a T58A variant of the C-MYC protein, an N-MYC protein or an L-MYC protein, a nucleic acid encoding any of the proteins, or a gene expression vector comprising said nucleic acid in a state that allows for the expression.

8. The agent for inducing iPS cells of claim 6, wherein said KLF1, KLF2, KLF4, or KLF5 protein comprises an amino acid substitution of the following (a) to (d):
(a) serine at position 349 and/or leucine at position 356 in the amino acid sequence of SEQ ID NO: 1;
(b) serine at position 342 and/or leucine at position 349 in the amino acid sequence of SEQ ID NO: 3;
(c) serine at position 500 and/or leucine at position 507 in the amino acid sequence of SEQ ID NO: 5; or
(d) serine at position 443 and/or leucine at position 450 in the amino acid sequence of SEQ ID NO: 7.

9. The agent for inducing iPS cells of claim 8, wherein:
said substitution of (a) is S349A, and/or L356A, L356N, L356D, L356C, L356E, L356G, L356K, L356M, L356S, or L356T,
said substitution of (b) is S342A, and/or L349A, L349N, L349D, L349C, L349E, L349G, L349K, L349M, L349S, or L349T,
said substitution of (c) is S500A, and/or L507A, L507N, L507D, L507C, L507E, L507G, L507K, L507M, L507S, or L507T, or
said substitution of (d) is S443A, and/or L450A, L450N, L450D, L450C, L450E, L450G, L450K, L450M, L450S, or L450T.

10. An agent for direct reprogramming, comprising any of the OCT3/4 protein variant or the peptide fragment thereof of claim 1, the nucleic acid of claim 3, or the gene expression vector of claim 4.

11. Use of the agent for inducing iPS cells of claim 5 in the production of iPS cells from somatic cells.

12. A method for producing iPS cells, comprising
an transduction step of transducing an agent for inducing iPS cells comprising the following (1) to (3), into somatic cells:
(1) any of the OCT3/4 protein variant or the peptide fragment thereof of claim 1, the nucleic acid of claim 3, or the gene expression vector of claim 4,
(2) any of a KLF1 protein, a KLF2 protein, a KLF4 protein, or a KLF5 protein, a nucleic acid encoding any of the proteins, or a gene expression vector comprising said nucleic acid in a state that allows for the expression, and
(3) any of a SOX1 protein, a SOX2 protein, a SOX3 protein, a SOX15 protein or a SOX17 protein, a nucleic acid encoding any of the proteins, or a gene expression vector comprising said nucleic acid in a state that allows for the expression; and
a culturing step of culturing the somatic cells after said transduction step in the presence of any one or more of a basic fibroblast growth factor, a TGF-β1 protein, a BMP protein, a Wnt3 protein, a GSK3β inhibitor, a Wnt inhibitor, retinoic acid, ascorbic acid, and a ROCK inhibitor.

13. A method for producing iPS cells, comprising
a transduction step of transducing an agent for inducing iPS cells comprising the following (1) to (4), into somatic cells:
(1) any of the OCT3/4 protein variant or the peptide fragment thereof of claim 1, the nucleic acid of claim 3, or the gene expression vector of claim 4,
(2) any of a KLF1 protein, a KLF2 protein, a KLF4 protein, or a KLF5 protein, a nucleic acid encoding any of the proteins, or a gene expression vector comprising said nucleic acid in a state that allows for the expression,
(3) any of a SOX1 protein, a SOX2 protein, a SOX3 protein, a SOX15 protein or a SOX17 protein, a nucleic acid encoding any of the proteins, or a gene expression vector comprising said nucleic acid in a state that allows for the expression, and
(4) any of a C-MYC protein, an N-MYC protein, an L-MYC protein, or a T58A variant protein of the C-MYC protein, a nucleic acid encoding any of the proteins, or a gene expression vector comprising said nucleic acid in a state that allows for the expression; and
a culturing step of culturing the somatic cells after said transduction step.

14. The method of claim 12, further comprising a selection step of selecting iPS cells transduced in said culturing step.

15. The method of claim 12, wherein said somatic cells are derived from a human.
